# FASCICULE DE BREVET EUROPEEN

(11) **EP 2 403 869 B1**
(45) Date de publication et mention de la délivrance du brevet: **08.05.2013**
(21) Numéro de dépôt: 10711431.6
(22) Date de dépôt: 05.03.2010
(51) Int. Cl.: A61K 38/17

(54) **Peptides utilisables pour le traitement des cancers et notamment de la leucemie lymphoïde chronique**
Peptide zur Behandlung von Krebs und insbesondere chronischer lymphoider Leukämie
Peptides used for treating cancers and, in particular, chronic lymphoid leukaemia

(30) Priorité: 05.03.2009 FR 0901007
(43) Date de publication de la demande: 11.01.2012
(73) Titulaire: Centre National de la Recherche Scientifique, 75016 Paris (FR); Centre Hospitalier Universitaire de Brest, 29200 Brest (FR); Université de Bretagne Occidentale, 29200 Brest (FR); Université Pierre et Marie Curie - Paris 6, 75005 Paris (FR)
(72) Inventeur: COSSON, Bertrand, F-29670 Taule (FR); SAAD, Hussam, F-29440 Treflaouenan (FR); CORMIER, Patrick, F-29233 Cleder (FR); BERTHOU, Christian, F-29200 Brest (FR); CZJZEK, Mirjam, F-29250 Plougoulm (FR)
(74) Mandataire: Marcadé, Véronique
(86) Numéro de dépôt international: PCT/FR2010/000191
(87) Numéro de publication internationale: WO 2010/100351

(56) Documents cités:
- WO-A1-2008/150689
- WO-A2-2007/150077
- GINGRAS AC ET AL: "eIF4 Interaction Factors: Effectors of mRNA Recruitment to Ribosomes and Regulators of Translation" ANNUAL REVIEW OF BIOCHEMISTRY, vol. 68, 1 juillet 1999 (1999-07-01), pages 913-963, XP002548577
- SAAD H; BOLLE R; MORALES J; COSSON B; MULNER-LORILLON O; BERTHOU C; CORMIER P: "Facteurs d'initiation eIF4: du developpement embryonnaire de l'oursin a la leucemie lymphoide chronique [Initiation factors eIF4: from sea urchin embryonic development to chronic lymphocytic leukemia]" JOURNAL DE LA SOCIETE DE BIOLOGIE, SOCIETE DE BIOLOGIE, PARIS, FR, vol. 201, no. 3, 1 janvier 2007 (2007-01-01), pages 307-315, XP008110663 ISSN: 1295-0661
- SCHMITT M ET AL: "RHAMM-R3 peptide vaccination in patients with acute myeloid leukemia, myelodysplastic syndrome, and multiple myeloma elicits immunologic and clinical responses" BLOOD, vol. 111, 31 juillet 2007 (2007-07-31), pages 1357-1365, XP002548578
- GREINER J ET AL: "High-Dose RHAMM-R3 Peptide Vaccination for Patients with Acute Myeloid Leukemia (AML), Myelodysplastic Syndrome (MDS), Multiple Myeloma (MM) and Chronic Lymphocytic Leukemia (CLL)" BLOOD (ASH ANNUAL MEETING ABSTRACTS), vol. 112, no. 11, 2911, 16 novembre 2008 (2008-11-16), page 1001, XP008112976

### SEQUENCE LISTING

<110> CENTRE NATIONAL DE LA RECHERCHE SCIENTIFIQUE
   CENTRE HOSPITALIER UNIVERSITAIRE DE BREST
   UNIVERSITE DE BRETAGNE OCCIDENTALE
   UNIVERSITE PIERRE ET MARIE CURIE (PARIS 6)
   COSSON, Bertrand
   SAAD, Hussam
   CORMIER, Patrick
   BERTHOU, Christian
   CZJZEK, Mirjam
<120> PEPTIDES UTILISABLES POUR LE TRAITEMENT DES CANCERS ET NOTAMMENT DE LA LEUCEMIE LYMPHOÏDE CHRONIQUE
<130> BLoclgF644/212
<160> 30
<170> PatentIn version 3.3
<210> 1
   <211> 12
   <212> PRT
   <213> Artificial
<220>
   <223> peptide
<220>
   <221> VARIANT
   <222> (1)..(1)
   <223> peut être remplacé par = "Lys" ou "His"
<220>
   <221> VARIANT
   <222> (2)..(2)
   <223> peut être remplacé par = "Lys" ou "His"
<220>
   <221> VARIANT
   <222> (3)..(3)
   <223> peut être remplacé par = "Arg" ou "His"
<220>
   <221> VARIANT
   <222> (5) .. (5)
   <223> peut être remplacé par n'importe quel acide aminé
<220>
   <221> VARIANT
   <222> (6) .. (6)
   <223> peut être remplacé par n'importe quel acide aminé
<220>
   <221> VARIANT
   <222> (7) .. (7)
   <223> peut être remplacé par n'importe quel acide aminé
<220>
   <221> VARIANT
   <222> (8) .. (8)
   <223> peut être remplacé par = "Ala" ou "Val" ou "Leu" ou "Ile" ou "Met"
<220>
   <221> VARIANT
   <222> (10)..(10)
   <223> peut être remplacé par = ""Met" ou "Phe"
<220>
   <221> VARIANT
   <222> (11)..(11)
   <223> peut être remplacé par n'importe quel acide aminé
<220>
   <221> VARIANT
   <222> (12)..(12)
   <223> peut être remplacé par = "Tyr" ou "Trp" ou "His"
<400> 1
   Arg Arg Lys Tyr Gly Arg Asp Phe Leu Leu Arg Phe
   1               5                   10
<210> 2
   <211> 17
   <212> PRT
   <213> Artificial
<220>
   <223> peptide
<400> 2
   Arg Arg Lys Tyr Gly Arg Asp Phe Leu Leu Arg Phe Arg Tyr Ile Arg
   1               5                   10                  15
   Ser
<210> 3
   <211> 12
   <212> PRT
   <213> Artificial
<220>
   <223> peptide
<400> 3
   Arg Arg Lys Tyr Gly Arg Asp Phe Leu Leu Arg Phe
   1               5                   10
<210> 4
   <211> 5
   <212> PRT
   <213> Artificial
<220>
   <223> peptide
<400> 4
   Arg Tyr Ile Arg Ser
   1 5
<210> 5
   <211> 12
   <212> PRT
   <213> Artificial
<220>
   <223> peptide
<400> 5
   Arg Arg Lys Ala Gly Arg Asp Phe Ala Ala Arg Phe
   1 5 10
<210> 6
   <211> 17
   <212> PRT
   <213> Artificial
<220>
   <223> peptide
<400> 6
   Arg Arg Lys Ala Gly Arg Asp Phe Ala Ala Arg Phe Arg Tyr Ile Arg
   1 5 10 15
   Ser
<210> 7
   <211> 17
   <212> PRT
   <213> Artificial
<220>
   <223> peptide
<400> 7
   Arg Ile Ile Tyr Asp Arg Lys Phe Leu Leu Asp Arg Arg Tyr Ile Arg
   1 5 10 15
   Ser
<210> 8
   <211> 17
   <212> PRT
   <213> Artificial
<220>
   <223> peptide
<400> 8
   Arg Ile Ile Ala Asp Arg Lys Phe Ala Ala Asp Arg Arg Tyr Ile Arg
   1 5 10 15
   Ser
<210> 9
   <211> 17
   <212> PRT
   <213> Artificial
<220>
   <223> peptide
<400> 9
   Arg Ile Ile Tyr Asp Arg His Phe Leu Leu Asn Met Arg Tyr Ile Arg
   1 5 10 15
   Ser
<210> 10
   <211> 17
   <212> PRT
   <213> Artificial
<220>
   <223> peptide
<400> 10
   Arg Ile Ile Ala Asp Arg His Phe Ala Ala Asn Met Arg Tyr Ile Arg
   1 5 10 15
   Ser
<210> 11
   <211> 17
   <212> PRT
   <213> Artificial
<220>
   <223> peptide
<400> 11
   Arg Arg Ile Tyr Gly Arg Asp Phe Leu Leu Arg Phe Arg Tyr Ile Arg
   1 5 10 15
   Ser
<210> 12
   <211> 15
   <212> PRT
   <213> Artificial
<220>
   <223> peptide
<400> 12
   Lys Tyr Gly Arg Asp Phe Leu Leu Arg Phe Arg Tyr Ile Arg Ser
   1 5 10 15
<210> 13
   <211> 17
   <212> PRT
   <213> Artificial
<220>
   <223> peptide
<400> 13
   Lys Lys Lys Tyr Gly Arg Asp Phe Leu Leu Arg Phe Arg Tyr Ile Arg
   1 5 10 15
   Ser
<210> 14
   <211> 17
   <212> PRT
   <213> Artificial
<220>
   <223> peptide
<400> 14
   Gly Gly Lys Tyr Gly Arg Asp Phe Leu Leu Arg Phe Arg Tyr Ile Arg
   1 5 10 15
   Ser
<210> 15
   <211> 17
   <212> PRT
   <213> Artificial
<220>
   <223> peptide
<400> 15
   Arg Arg Arg Tyr Gly Arg Asp Phe Leu Leu Arg Phe Arg Tyr Ile Arg
   1 5 10 15
   Ser
<210> 16
   <211> 17
   <212> PRT
   <213> Artificial
<220>
   <223> peptide
<400> 16
   Arg Arg Lys Tyr Ala Arg Asp Phe Leu Leu Arg Phe Arg Tyr Ile Arg
   1 5 10 15
   Ser
<210> 17
   <211> 17
   <212> PRT
   <213> Artificial
<220>
   <223> peptide
<400> 17
   Arg Arg Lys Tyr Ser Arg Asp Phe Leu Leu Arg Phe Arg Tyr Ile Arg
   1 5 10 15
   Ser
<210> 18
   <211> 17
   <212> PRT
   <213> Artificial
<220>
   <223> peptide
<400> 18
   Arg Arg Lys Tyr Gly Lys Asp Phe Leu Leu Arg Phe Arg Tyr Ile Arg
   1 5 10 15
   Ser
<210> 19
   <211> 17
   <212> PRT
   <213> Artificial
<220>
   <223> peptide
<400> 19
   Arg Arg Lys Tyr Gly His Asp Phe Leu Leu Arg Phe Arg Tyr Ile Arg
   1 5 10 15
   Ser
<210> 20
   <211> 17
   <212> PRT
   <213> Artificial
<220>
   <223> peptide
<400> 20
   Arg Arg Lys Tyr Gly Arg Glu Phe Leu Leu Arg Phe Arg Tyr Ile Arg
   1 5 10 15
   Ser
<210> 21
   <211> 17
   <212> PRT
   <213> Artificial
<220>
   <223> peptide
<400> 21
   Arg Arg Lys Tyr Gly Arg Ala Phe Leu Leu Arg Phe Arg Tyr Ile Arg
   1 5 10 15
   Ser
<210> 22
   <211> 17
   <212> PRT
   <213> Artificial
<220>
   <223> peptide
<400> 22
   Arg Arg Lys Tyr Gly Arg Asp Phe Leu Leu Arg Lys Arg Tyr Ile Arg
   1 5 10 15
   Ser
<210> 23
   <211> 17
   <212> PRT
   <213> Artificial
<220>
   <223> peptide
<400> 23
   Arg Arg Lys Tyr Gly Arg Asp Phe Leu Leu Arg Trp Arg Tyr Ile Arg
   1 5 10 15
   Ser
<210> 24
   <211> 17
   <212> PRT
   <213> Artificial
<220>
   <223> peptide
<400> 24
   Arg Ile Lys Tyr Asp Arg Lys Phe Leu Leu Asp Arg Arg Tyr Ile Arg
   1 5 10 15
   Ser
<210> 25
   <211> 7
   <212> PRT
   <213> Artificial
<220>
   <223> peptide
<220>
   <221> VARIANT
   <222> (2)..(2)
   <223> peut être remplacé par n'importe quel autre acide aminé
<220>
   <221> VARIANT
   <222> (3)..(3)
   <223> peut être remplacé par n'importe quel autre acide aminé
<220>
   <221> VARIANT
   <222> (4)..(4)
   <223> peut être remplacé par n'importe quel autre acide aminé
<220>
   <221> VARIANT
   <222> (5)..(5)
   <223> peut être remplacé par n'importe quel autre acide aminé
<220>
   <221> VARIANT
   <222> (7)..(7) remplacé par = "Met" ou "Phe"
   <223> peut être remplacé par = "Met" ou "Phe"
<400> 25
   Tyr Gly Arg Asp Phe Leu Leu
   1               5
<210> 26
   <211> 54
   <212> DNA
   <213> Artificial
<220>
   <223> amorce
<400> 26
   taattgctag cttaatacga ctcactatag ggaccggtcg ccaccatggt gagc 54
<210> 27
   <211> 34
   <212> DNA
   <213> Artificial
<220>
   <223> amorce
<400> 27
   ttattgaatt cacttgtaca gctcgtccat gccg 34
<210> 28
   <211> 88
   <212> DNA
   <213> Artificial
<220>
   <223> amorce
<400> 28
   ttattgaatt caagcgatgc tgcagaagcg gaaacgtagc aggaagtctc ggccatactt 60
   gcgtctcttg tacagctcgt ccatgccg 88
<210> 29
   <211> 42
   <212> DNA
   <213> Artificial
<220>
   <223> amorce
<400> 29
   gctgtacaag agacgcaagg ctggccgaga cttcctgcta cg 42
<210> 30
   <211> 17
   <212> PRT
   <213> Artificial
<220>
   <223> peptide
<400> 30
   Arg Arg Lys Tyr Ala Arg Ala Phe Leu Leu Arg Phe Arg Tyr Ile Arg
   1               5                   10                  15
   Ser

## Description

La leucémie lymphoïde chronique (LLC) est une hémopathie définie par l'accumulation d'un clone de petits lymphocytes B dans le sang (lymphocytes supérieurs à 4.10⁹/Litre) et la moelle osseuse. La LLC est la plus fréquente des hémopathies chez les adultes en occident (O'Brien et al., 1995). Elle représente le tiers de l'ensemble des leucémies. Son incidence atteint 2,7 pour 10⁵ individus aux États-Unis et en Europe. Cette incidence atteint 20 pour 10⁵ pour les personnes âgées de plus de 70 ans. La découverte de la maladie est fortuite dans la majorité des cas, à l'occasion d'un hémogramme chez un adulte en bonne santé apparente, conduisant à la découverte d'une hyper lymphocytose variable, habituellement comprise entre 5 et 50 000/mm³ et atteignant parfois des centaines de milliers de lymphocytes B. Ces lymphocytes B présentent des caractéristiques particulières qui permettent de les distinguer des lymphocytes B normaux..

Les Lymphocytes B de la LLC expriment des immunoglobulines à leur surface avec une monotypie des chaînes légères (Preud'homme and Seligmann, 1972) et des déterminants idiotypiques des chaînes lourdes (Fu et al., 1975), témoignant de la nature monoclonale de la cellule. La mono clonalité est confirmée par l'étude du réarrangement des gènes des immunoglobulines (Fialkow et al., 1978).

Les LLC sont caractérisées par une expression quasiment constante du marqueur CD5 (Boumsell et al., 1978). Le marqueur CD5 est habituellement présent sur les cellules T mûres et est exprimé dans une sous-population des cellules B normales. Ces caractéristiques phénotypiques constituent aujourd'hui un des critères diagnostic de la maladie (Moreau et al., 1997).

Le diagnostic repose sur la présence d'une lymphocytose excessive constatée sur plusieurs hémogrammes d'aspect morphologique particulier ; l'étude immunologique confirme l'augmentation d'une population de lymphocytes B monoclonaux CD19+ CD5+ CD23+ (Caligaris-Cappio and Hamblin, 1999).

L'activité proliférative des lymphocytes B de la LLC est faible. En effet, comme le montre l'observation de l'expression des régulateurs du cycle cellulaire, les cellules sont bloquées en phase G0/G1 du cycle cellulaire (Delmer et al., 1995). L'accumulation clonale de ces cellules est liée à un défaut d'un mécanisme de mort cellulaire programmée (*programmed cell death, PCD)* appelé apoptose.

La protéine p27 est un inhibiteur de prolifération, dont la faible expression est souvent associée à un mauvais pronostic dans le cas de tumeur proliférative. Par contre, pour la LLC qui n'est pas une tumeur proliférative, c'est la surexpression de p27 qui est un facteur pronostique défavorable, et pourrait influencer les processus d'apoptose (Vrhovac et al., 1998).

eIF4E (eukaryotic Initiation Factor 4E) est un facteur déterminant dans le contrôle de l'initiation de la traduction dépendante de la coiffe. eIF4E se lie aux ARNm possédant une coiffe (appelée "cap") à l'extrémité 5', et s'associe à eIF4G pour stimuler la synthèse protéique. L'inhibition compétitive de cette interaction par l'association de eIF4E avec 4EBP *(eIF4E Binding Protein)* est un mécanisme de régulation de l'initiation de la traduction essentiel, en particulier dans l'ovule d'oursin. Il a été démontré chez l'oursin que l'augmentation de l'activité traductionnelle à la fécondation passe par une dissociation du complexe eIF4E-4EBP et la dégradation de 4E-BP (Cormier et al., 2003). La traduction d'ARNm spécifiques peut également être régulée par des protéines interagissant à la fois avec eIF4E et avec l'ARNm (pour revue voir (Richter and Sonenberg, 2005)).

Le rôle d'eIF4E dans la régulation de l'expression des ARNm ne se limite pas à l'initiation de la traduction. La liaison d'eIF4E à la coiffe des ARNm les protège d'une dégradation exonucléolytique. L'interaction d'eIF4E avec ses partenaires induit des modifications conformationnelles, ce qui change son affinité pour la coiffe et peut conduire à la dégradation de l'ARNm. L'interaction d'eIF4E à 4E-T est impliquée dans le routage cellulaire des ARNm vers des lieux de stockage et de dégradation.

Les protéines eIF4E et 4EBP sont impliquées dans les mécanismes de survie et d'apoptose : eIF4E est caractérisé comme étant anti-apoptotique et inversement, 4EBP est identifié comme pro-apoptotique (Holcik and Sonenberg, 2005; Morley et al., 2005)).

Récemment, une nouvelle fonction a été décrite : eIF4E régule l'export nucléo-cytoplasmique d'ARNm comportant une séquence 4E-SE (*4E sensitivity element*)*.* Certains ARNm comportant cette séquence codent pour des protéines impliquées dans la régulation du cycle cellulaire, ce qui donne un éclairage nouveau sur les propriétés prolifératives et oncogéniques d'eIF4E. Cette fonction d'eIF4E requiert son interaction avec différents partenaires dont des protéines à homéodomaines comme HoxA9 (Culjkovic et al., 2005; Topisirovic et al., 2005).

La traduction de la majorité des ARNm codant pour des protéines impliquées dans la croissance et la prolifération cellulaire est fortement dépendante de la disponibilité d'eIF4E pour former un complexe fonctionnel avec eIF4G dans la cellule (Koromilas et al., 1992). 4EBP régule négativement cette interaction en s'associant à eIF4E. Dans le but de mimer la fonction de 4EBP, des peptides ou des molécule synthétiques capable de se lier à eIF4E ont été recherchées (Herbert et al., 2000; Moerke et al., 2007).

Une librairie de composés chimiques a été criblée sur la propriété de ces composés à se lier à eIF4E (Moerke et al., 2007). Un composé sélectionné, 4EGI-1, est capable d'inhiber la traduction de protéines impliquées dans la croissance et la prolifération cellulaire, cet effet étant plus important dans les cellules qui surexpriment l'oncogène bcr-abl. De plus, 4EGI-1 provoque une augmentation de la proportion d'ADN en sub-G0/G1, et l'apparition d'une morphologie nucléaire de type apoptotique. 4EGI-1 n'est plus actif en présence de ZVAD.fmk, un inhibiteur de l'apoptose. 4EGI-1 entraîne donc une PCD de type apoptotique. Ces expériences ayant été réalisées sur des cellules en présence de sérum, l'effet de 4EGI-1 s'exerce donc sur des cellules en prolifération, et passe par la voie d'apoptose "classique".

L'introduction de peptides basés sur le motif de liaison de 4EBP et eIF4G dans les cellules MRC5, cultivées sans sérum pendant 72 heures, conduit à une mort cellulaire rapide (Herbert et al., 2000). Cette mort cellulaire s'apparente à une PCD, d'une part par les caractéristiques de l'ADN des cellules traitées (fragmentation observée en immunofluorescence, profil sub-G0/G1en FACS) et d'autre part par une modification de la perméabilité mitochondriale. Cette PCD n'est pas l'apoptose "classique" puisqu'elle est indépendante de l'activation de caspases et n'est pas inhibée par l'inhibiteur de l'apoptose ZVAD.fmk. De plus, cette mort cellulaire est indépendante de la traduction puisque l'effet des peptides perdure après traitement des cellules par des inhibiteurs de la traduction. Contrairement à l'étude de Moerke (*supra*)*,* l'effet des peptides s'exerce sur des cellules cultivées sans sérum, donc sur des cellules arrêtées en GO/G1, et par un mécanisme de PCD différent de l'apoptose "classique".

Dans ce contexte, les inventeurs ont évalué la capacité de peptides pouvant interagir avec eIF4E, connus ou identifiés par une approche de génomique fonctionnelle originale, à induire la mort cellulaire des lymphocytes LLC. Ils ont ainsi mis en évidence que contrairement aux peptides décrits dans l'art antérieur pour leur capacité à se lier à eIF4E, le peptide A1 WT IRS, de séquence RRKYGRDFLLRFRYIRS (SEQ ID No : 2), lorsqu'il est incubé avec des lymphocytes B issus de patients atteints de LLC et mis en culture, provoque une forte mortalité de ces lymphocytes. Cet effet est essentiellement précoce au cours de l'incubation avec les cellules en culture, et présente une spécificité intéressante vis-à-vis des lignées cellulaires myéloïdes et lymphoïdes. Ainsi, de manière particulièrement avantageuse, le peptide de l'invention provoque la mort cellulaire de lignées lymphocytaires dès 15 minutes après la mise en contact du peptide et des cellules, ce qui en fait un composé à action particulièrement rapide par rapport aux composés de l'état de la technique connus pour induire la mort cellulaire. Une telle rapidité d'action permet d'envisager une limitation les mécanismes de résistance qui peuvent être induits dans l'organisme en réponse à un traitement thérapeutique à l'aide de composés à action plus lente.

Les inventeurs ont également pu mettre en évidence que les effets d'induction de la mort cellulaire du peptide A1 WT pouvaient être étendus à des lignées cellulaires issues de tumeurs solides.

Le peptide A1 WT IRS correspond à une séquence issue de la protéine *Angel 1* humaine (peptide A1 WT, de séquence RRKYGRDFLLRF (SEQ ID No : 3)), fusionnée à son extrémité C-terminale à une séquence favorisant son entrée dans les cellules. Les inventeurs ont émis l'hypothèse que ce peptide pouvait conserver son activité malgré plusieurs substitutions dans sa séquence, déterminant ainsi une séquence générique de peptides actifs dans ce contexte.

L'invention porte donc en premier lieu sur un peptide comprenant la séquence X₁X₂X₃YX₄X₅X₆X₇LX₈X₉X₁₀ (SEQ ID No : 1), dans laquelle :
- X₁ et X₂ représentent, indépendamment l'un de l'autre, un acide aminé choisi parmi R, K et H, avec de préférence X₁ = X₂ =R ;
- X₃ représente un acide aminé choisi parmi R, K et H, de préférence R ou K;
- X₄, X₅ et X₆ représentent n'importe quel acide aminé, avec de préférence :
   - X₄ = G, A ou S, plus préférentiellement A ou S, et/ou
   - X₅ = R ou H, plus préférentiellement R, et/ou
   - X₆ = D, E, A, V, L, P, M, F, I ou X, plus préférentiellement D ou A ;
- X₇ représente un acide aminé choisi parmi F, A, V, L, I, M, de préférence F;
- X₈ représente un acide aminé choisi parmi L, M et F, de préférence L ;
- X₉ représente n'importe quel acide aminé, de préférence R ;
- X₁₀ représente un acide aminé choisi parmi F, Y, W et H, de préférence F ou W,
pour le traitement d'une maladie hématopoïétique maligne.

Le peptide selon l'invention sera particulièrement efficace pour traiter une leucémie ou un lymphome, et notamment la leucémie lymphoïde chronique (LLC), la leucémie prolymphocytaire B (LPLB), la leucémie aigüe lymphoblastique T (LALT), le lymphome de la zone marginale (LZM), le lymphome du manteau et la leucémie aigüe myéloïde (LAM).

Dans le présent texte, le terme "peptide" désigne n'importe quelle molécule dont le coeur est constitué d'acides aminés, en configuration L ou D, qui peuvent éventuellement être remplacés par des acides aminés modifiés pour avoir de meilleures propriétés pharmacocinétiques, tels que des acides α-aminés N-méthylés ou des acides aminés (éventuellement non protéinogéniques) contraints (Cowell et al., 2004). Dans un peptide au sens de la présente invention, les acides aminés ou leurs analogues peuvent être reliés entre eux par des liaisons peptidiques (-CO-NH-), mais le terme "peptide" désigne également tout enchaînement d'acides aminés (ou analogues) dans lequel une ou plusieurs liaisons peptidiques ont été modifiées, notamment pour augmenter la résistance de la molécule à la protéolyse. Le cas échéant, d'autres groupes chimiques peuvent être liés de façon covalente à ce peptide, pour lui procurer une meilleure stabilité, le protéger *in vivo* et/ou pour lui apporter des propriétés supplémentaires, par exemple pour favoriser son entrée dans les cellules, et/ou favoriser son adressage vers les cellules à traiter (dans le cas de la LLC, les lymphocytes B).

A titre d'exemples de groupes susceptibles d'être liés au peptide de l'invention pour améliorer sa stabilité et/ou ralentir sa destruction ou son élimination *in vivo,* on peut citer les groupes cystéamide, cystéine, thiol, amide, carboxyles, alkyles en C₁-C₆ linéaires ou ramifiés, alkyles substitués, amines primaires ou secondaires, dérivés osidiques, lipides, phospholipides, acides gras, cholestérol, poly-éthylène glycol, acétyle, etc. L'homme du métier pourra choisir de greffer, aux extrémités N et/ou C-terminales du peptide, un ou plusieurs de ces groupes. Si nécessaire, par exemple dans le cas d'une addition N-terminale de cholestérol, un pont peptidique sera utilisé pour lier une molécule non peptidique au peptide de l'invention. Un exemple de pont utilisable pour cela est le pont -CA_{β}-.

Un autre moyen, largement illustré par la littérature scientifique, d'obtenir une protection et une stabilisation des *peptides in vivo* est de les cycliser. Le cas échéant, un pont, peptidique ou autre (par exemple S-S ou C-C), est ajouté entre les extrémités N- et C-terminales du peptide linéaire, afin d'assurer sa cyclisation. Selon un mode de réalisation préféré de l'invention, le peptide comprenant la séquence SEQ ID No : 1 est cyclisé.

A titre d'exemples de groupes favorisant l'entrée du peptide de l'invention dans les cellules, on peut citer certaines séquences peptidiques, par exemple les séquences dites "pénétratines" telles que la séquence IRS composée de la séquence d'acides aminés RYIRS (SEQ ID No : 4) (utilisée dans la partie expérimentale présentée ci-dessous), ou la séquence KKWKMRRNQFWVKVQRG (Kanovsky et al., 2001). Une telle séquence sera de préférence liée à l'extrémité C-terminale du peptide de l'invention, par une liaison peptidique ou autre. D'autres éléments susceptibles de favoriser l'entrée du peptide dans la cellule peuvent être utilisés, comme par exemple des nanoparticules.

A titre d'exemples de groupes favorisant l'adressage du peptide vers certains types cellulaires, on peut citer, bien entendu, des composés peptidiques tels que des immunoglobulines, notamment des anticorps, ainsi que des épitopes ou des fragments d'anticorps (Fab) ciblant spécifiquement des éléments de la surface cellulaire (antigènes, récepteurs) du type cellulaire ciblé, *etc.,* mais également des sucres, tels que des monosaccharides (ex : glucose, galactose, glucosamine ou galactosamine), des oligosaccharides, polysaccharides, ou leurs analogues, ainsi que certains oligonucléotides, ou certaines molécules organiques telles que le folate. La fonction d'adressage de telles molécules est liée au fait qu'elles constituent des ligands de certains récepteurs sur-exprimés à la surface cellulaire des cellules d'intérêt.

En particulier, l'adressage du peptide vers les lumphocytes B de LLC peut être assuré (i) par liaison à un peptide d'adressage tel que la séquence IRS mentionnée plus haut ; (ii) par liaison du peptide à une structure carbohydrate telle que le Sia-α2-6 Gal-β1-4G1cNAc (Hanasaki et al., 1995) qui cible la molécule CD22 des lymphocytes B ; et/ou (iii) par l'incorporation du peptide dans des immuno-nanoparticules recouvertes d'anticorps spécifique reconnaissant un ou des antigène(s) des lymphocytes B (ex :CD5 et HLA-DR ou CD5 -Fc γ RuB).

Il est à noter que la séquence IRS peut :
- soit être conservée,
- soit remplacée par un autre peptide d'adressage reconnaissant un récepteur d'endocytose sur le lymphocyte B de LLC (exp : HLA-DR),
- soit être supprimée si le peptide est lié à une structure carbohydrate ou si le peptide est incorporé dans une immuno-nanoparticule.

Les stratégies d'adressage peuvent également favoriser l'entrée du peptide dans les cellules.

Il est important de noter que bien que certains des éléments qui peuvent être liés au peptide de SEQ ID No : 1 comportent des acides aminés, l'invention ne concerne pas n'importe quel peptide ou protéine comprenant cette séquence, indépendamment des acides aminés situés autour de la séquence SEQ ID No : 1. En effet, les seules séquences peptidiques qui peuvent être liées au peptide de l'invention sont, comme décrit ci-dessus, des ponts peptidiques (dont la longueur n'excédera pas 5 acides aminés), des peptides favorisant l'entrée dans les cellules (dont la longueur n'excédera pas 20 à 25 acides aminés, de préférence 5 à 17 acides aminés), ainsi que des peptides de ciblage (dont la taille n'excédera pas 50 acides aminés, de préférence 5 à 20 acides aminés). Un peptide selon l'invention possède donc des caractéristiques structurales (présence de la SEQ ID No : 1 ; taille n'excédant pas 75 acides aminés environ, de préférence moins de 20 acides aminés), mais également fonctionnelles, puisqu'il doit conserver l'activité du peptide A1 IRS de SEQ ID No : 2, telle que décrite à l'exemple 4 ci-dessous, le cas échéant combinée avec des fonctionnalités additionnelles telles que le ciblage de certains types cellulaires, notamment les lymphocytes B.

Selon une mise en oeuvre préférée de l'invention, le peptide utilisé pour le traitement d'une maladie telle que la leucémie lymphoïde chronique est le peptide A1 WT IRS de séquence RRKYGRDFLLRFRYIRS (Seq ID No : 2).

L'invention porte également sur une composition pharmaceutique, destinée notamment au traitement de la leucémie lymphoïde chronique, et comprenant un peptide tel que décrit ci-dessus, associé à un vecteur. Il existe une très abondante littérature sur les vecteurs utilisables pour délivrer des composés peptidiques dans des cellules, et l'homme du métier est libre, pour mettre en oeuvre l'invention, d'utiliser un peptide de séquence SEQ ID No :1 en combinaison avec n'importe quel élément susceptible de favoriser sa stabilité, son entrée dans les cellules et/ou son adressage. A titre d'exemples non limitatifs de vecteurs qui peuvent être utilisés pour délivrer le peptide de l'invention, on peut citer ceux décrits dans les articles de (Deshayes et al., 2008; Foged and Nielsen, 2008; Malik et al., 2007) et (Soares et al., 2007). Par ailleurs, le vecteur peut être une immunoglobuline, notamment un anticorps.

Outre les dispositions qui précèdent, l'invention comprend encore d'autres dispositions, qui ressortiront des exemples expérimentaux ci-dessous, et des figures annexées.

### Légendes des figures :

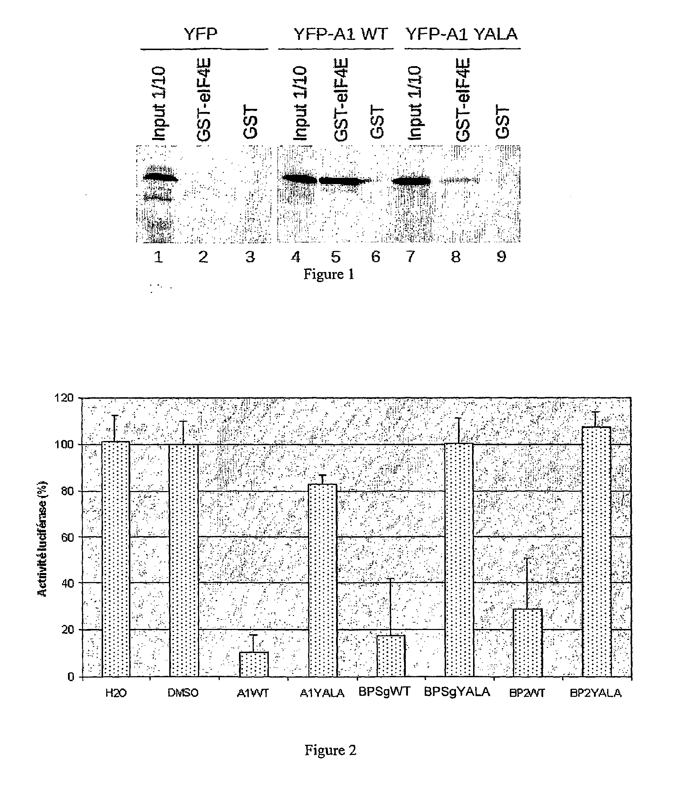

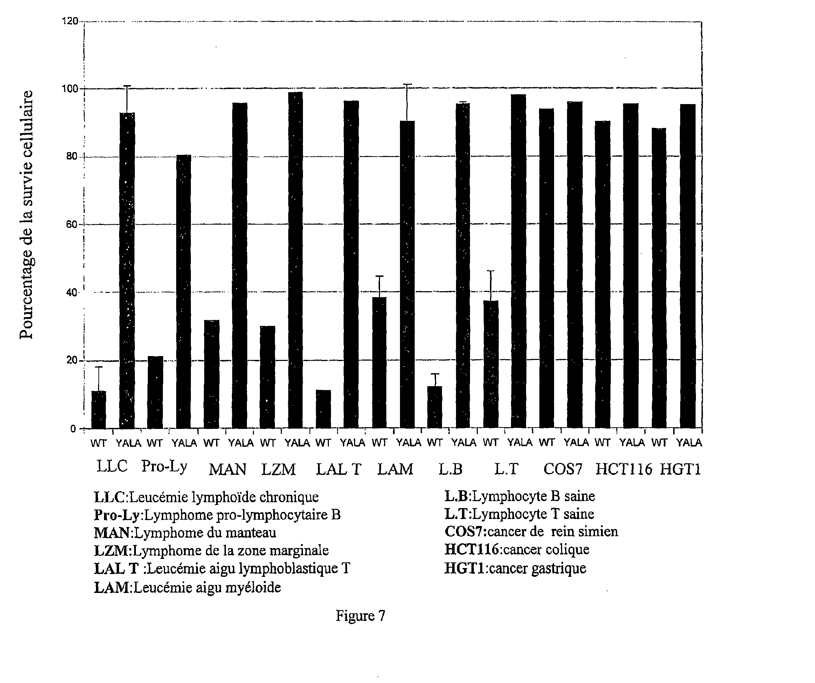

Figure 1 : Test d'interaction des séquences peptidiques à eIF4E
   La protéine eIF4E fusionnée à la glutathione-S-transférase (GST-eIF4E) ou la GST seule (GST) utilisée comme contrôle, sont produites en bactérie, purifiées et immobilisées sur des billes glutathione. Les protéines YFP, YFP-A1 WT et YFP-A1 YALA ont été produites en lysat de réticulocytes de lapin en présence de méthionine 35S et chromatographiées sur les billes GST-eIF4E ou GST. Les protéines retenues sur les billes sont éluées et analysées par SDS-PAGE suivie d'une autoradiographie.
   Un dixième des protéines YFP, YFP-A1 WT et YFP-A1 YALA utilisées par essai ont été chargées sur le gel (input 1/10, piste 1, 4, 7).
Figure 2 : Effet des peptides sur la traduction
   Les peptides possédant le motif IRS et les séquences de type 4EBPlike (A1WT, BPSgWT, et BP2WT) ou les séquences de type 4EBPlike mutantes (A1YALA, BPSgYALA, BP2YALA), sont incubés en lysat de réticulocyte de lapin contenant des ARNm coiffés codant pour la luciférase, enzyme dont l'activité est facilement dosable. L'activité traductionnelle est directement proportionnelle à l'activité de la luciférase produite. L'activité traductionnelle en présence de peptide est comparée à celle obtenue lors de l'ajout d'un volume équivalent de DMSO, considérée égale à 100%.

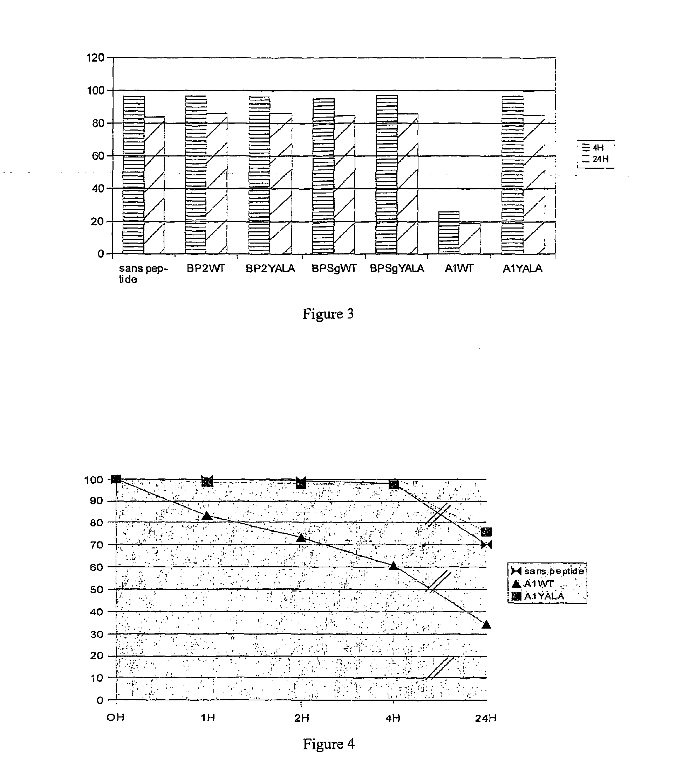

Figure 3 : Effet des peptides sur la survie cellulaire
   Des lymphocytes B de sang périphérique prélevé sur des patients atteints de la LLC puis purifiés sur coussin de Ficoll et mis en culture sont incubés en présence de 40µM des peptides décrits figure 2. Le pourcentage de survie cellulaire après 4 heures (colonne grise) ou 24 heures (colonne blanche) de culture correspond au pourcentage de cellules non marquées ni par l'annexine ni par l'iodure de propidium évalué par cytométrie de flux.
Figure 4 : Effet des peptides sur la survie des cellules après différents temps d'incubation :
   Les cellules sont traitées comme indiqué figure 3, sauf que le pourcentage de survie cellulaire est évalué après différent temps d'incubation :1H, 2H, 4H et 24H.
Figure 5 : Effet dose-dépendant du peptide A1WT sur la survie des cellules
   Les cellules sont traitées comme indiqué figure 3, avec une concentration de peptides de 0, 5, 10, 20, 30, ou 40 µM. La survie cellulaire est déterminée après 24H de culture en présence de peptide.
Figure 6 : Activité du peptide A1WT en présence d'émétine
   Les cellules sont préparées et mises en culture comme indiqué figure 3. Les cellules sont préincubées pendant une heure avec 100µM d'émétine avant ajout de 40µM des peptides A1WT ou A 1 YALA. Le pourcentage de survie cellulaire est évalué après 1H ,2H et 4H d'incubation.
Figure 7: Activité du peptide A1WT sur des cellules d'origine lymphocytaire, lymphoïde, gastrique, colique ou rénale.
   Les cellules sont préparées et mises en culture (voir Matériel et Méthodes) puis incubées pendant 4H avec le peptide A1WT ou A1 YALA. Le pourcentage de survie cellulaire correspond au pourcentage de cellules non marquées ni par l'annexine ni par l'iodure de propidium évalué par cytométrie de flux. Les noms des pathologies dont sont atteints les patients prélevés sont indiquées sous la figure. Les lymphocytes T (L.T) et B (L.B.) contrôle sont prélevés chez des patients souffrant d'une maladie non liée à une pathologie lymphocytaire (hémochromatose).

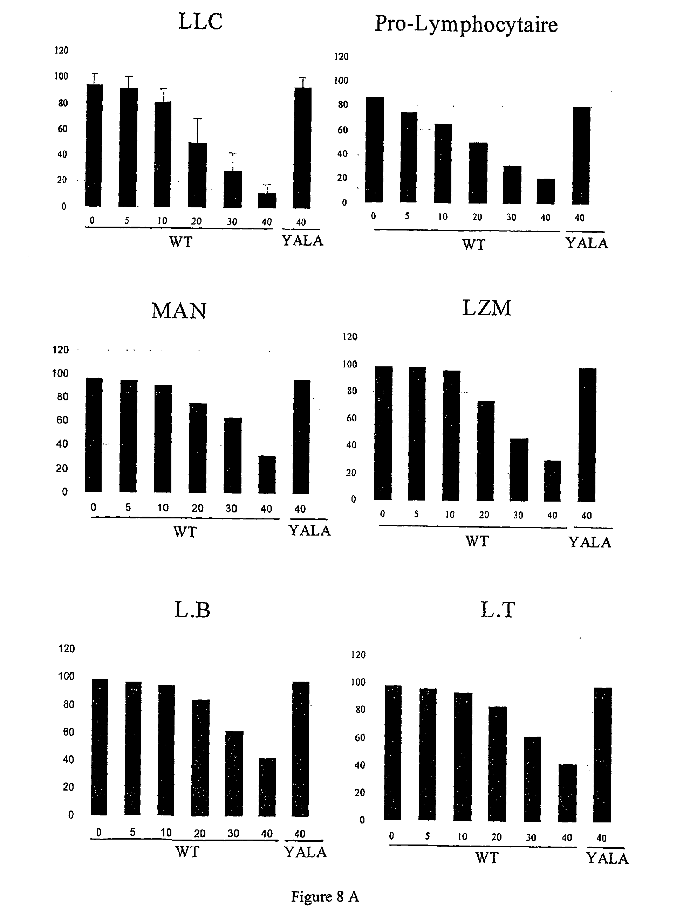

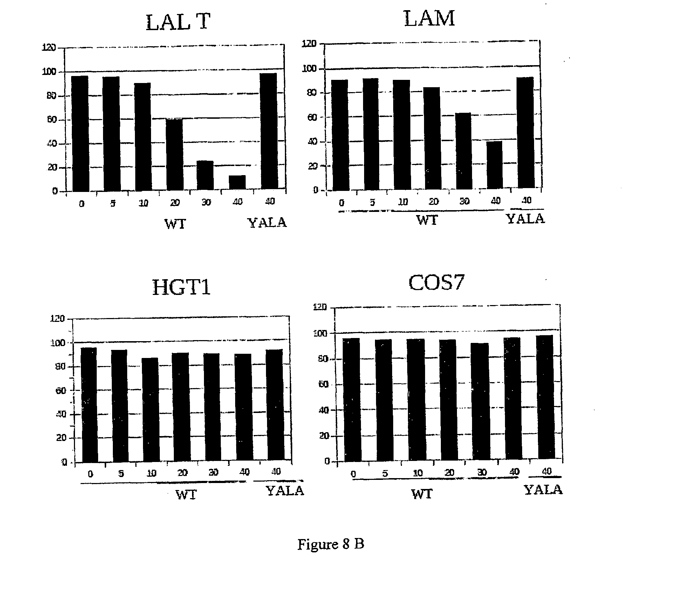

Figure 8 : Effet dose-dépendant du peptide A1WT sur des cellules d'origine lymphocytaire, lymphoïde, gastrique, colique ou rénale.
   Panel A et B, les cellules sont préparées et traitées comme indiqué figure 3, sauf que les cellules ont été incubées avec 0, 5, 10, 20, 30 ou 40 mM de peptide A1WT ou 40 mM de peptide A1 YALA. Les abréviations des noms des pathologies dont sont atteints les patients prélevés sont indiquées figure 7.

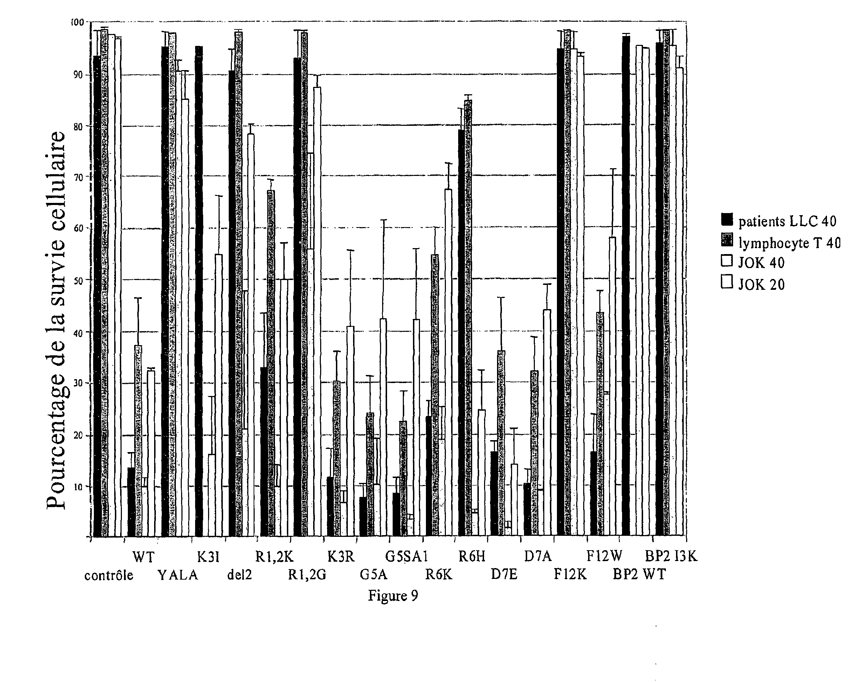

Figure 9 : Activité du peptide A1 WT IRS et de ses dérivés sur la survie des cellules LLC, lymphocytes T contrôles et Jok.
   Les cellules sont traitées comme indiqué figure 7, avec les cellules LLC, lymphocytes T contrôle et Jok, qui sont incubées avec le peptide A1 WT IRS et ses dérivés décrits dans le tableau 1. Les cellules Jok ont été incubées soit avec 20 µM (JOK 20) soit avec 40 µM (JOK 40) de chacun des peptides. Par souci de simplification de la légende le peptide A1 WT IRS est nommé « WT ». De même ses dérivés sont nommés par le nom des mutations (A1 KSI IRS est nommé « K3I » par exemple).

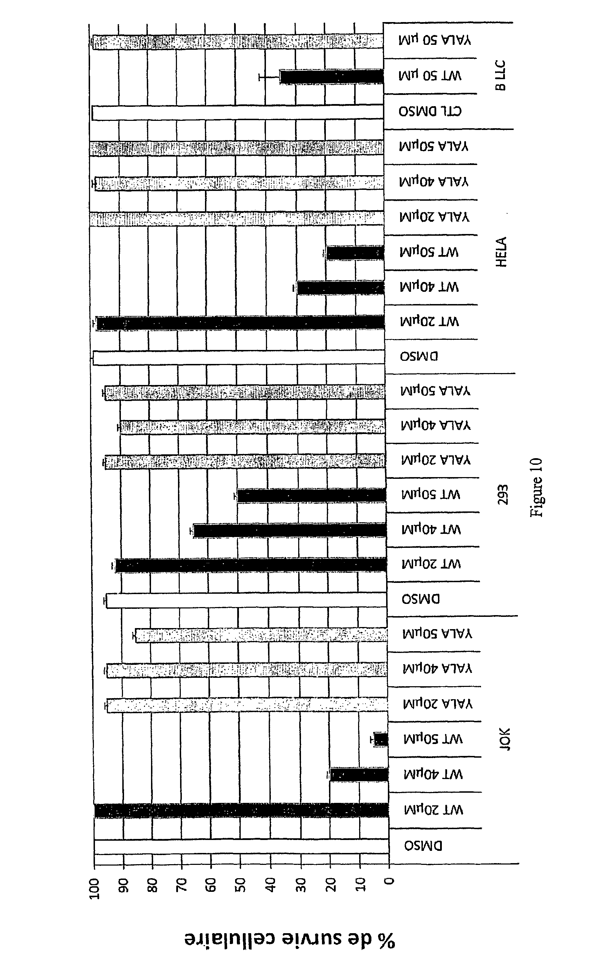

Figure 10 : Activité du peptide A1WT sur des cellules d'origine lymphocytaire (JOK et B LLC), rénale (293), et du col de l'utérus (HeLa).
   Les cellules cultivées en plaques multipuits à fond de verre sont incubées pendant une heure avec le peptide A1WT ou A1 YALA aux concentrations indiquées sur la figure. Le pourcentage de survie cellulaire est obtenu en calculant le rapport entre les cellules colorées par le Syto 13 et celles colorées par l'iodure de propidium (voir Matériel et Methodes).

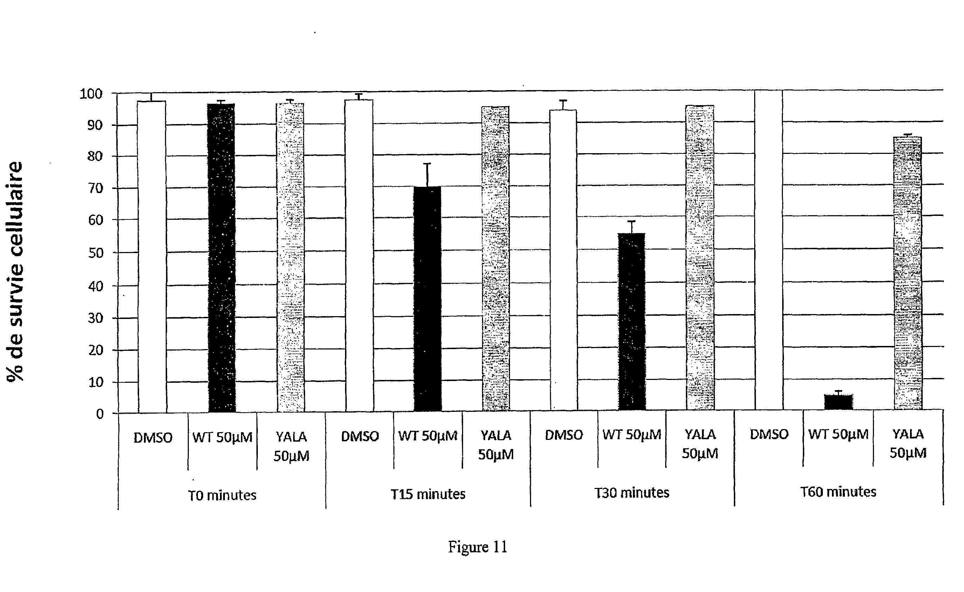

Figure 11 : Le peptide A1WT provoque une mortalité rapide des cellules d'origine lymphocytaire (JOK).
   Les cellules sont traitées comme indiqué figure 10 sauf que le temps d'incubation en présence du peptide varie de 15 mn à 1h.

### Exemples

Les exemples expérimentaux décrits ci-après ont été obtenus en utilisant les matériels et méthodes suivants :

### Test d'interaction des peptides fusionnés à YFP

Les plasmides codant pour les protéines YFP - peptides ont été obtenus par amplification du cadre de lecture de la protéine YFP (*enhanced yellow-green variant of the Aequorea victoria green fluorescent protein*) à partir du plasmide pEYFP-C1 (Clontech). L'amorce 5' utilisée (séquence décrite ci dessous) a permis d'insérer un promoteur T7 (séquence soulignée) en amont du cadre de lecture de YFP. L'amorce 3' YFP a assuré l'amplification de YFP alors que l'amorce 3' A1 WT a permis de fusionner l'extrémité C terminale de YFP à la séquence du peptide A1 WT. Les amplimères ont été clonés dans le plasmide pCR 2.1-TOPO (TOPO TA clonig kit, Invitrogen). La mutation de YFP-A1 WT pour obtenir YFP A1 YALA a été réalisée à l'aide de l'amorce de mutagénène A1 YALA. Les protéines YFP, YFP A1 WT et YFP A1 YALA ont été produites en lysat de réticulocyte de lapin (RRL) en présence Met 35S suivant les indications du fournisseur (TNT *T7 coupled rabbit reticulocyte lysate system,* Promega). Le plasmide pGEX 4E a été obtenu en insérant le cadre de lecture de la protéine eIF4E de souris au niveau des sites BamHI/EcoRI du vecteur PGEX-4T-1 (Amersham pharmacia biotech).La protéine recombinante eIF4E de souris fusionnée en N terminale à la GST a été produite en bactéries BL21DE3 (Novagen) à partir du plasmide pGEX4T-eIF4E et fixée sur des billes glutathion suivant les indications du fournisseur *(Glutathione* sepharose 4B bead column, Amersham Pharmacia Biotech). 5 ml de RRL programmé ont été incubés en présence de 20 ml de billes GST-eIF4E dans un volume de 400 ml de PBS. Après lavage, les protéines ont été éluées par du tampon de charge SDS, et analysées après gel SDS-PAGE par autoradiographie *(Typhoon* PhosphoImager, Amersham Pharmacia Biotech).
Amorce 5' :
Amorce 3' YFP :
   TTATTGAATTCACTTGTACAGCTCGTCCATGCCG (SEQ ID No : 27)
Amorce 3' A1 WT :
Amorce de mutagénèse A1 YALA :
   GCTGTACAAGAGACGCAAGGCTGGCCGAGACTTCCTGCTACG (SEQ ID No : 29)

### Analyse de l'effet des peptides sur la traduction en lysat de réticulocyte de lapin

Les peptides ont été produits, purifiés par HPLC (pureté >95%), et lyophylisés par Thermofisher Scientific. Les peptides ont ensuite été dissous à 10 mM dans du DMSO.

Les ARNm coiffés codant pour la luciférase Renillia (Luc R) ont été produits par le système *T7 Message* Machine Kit (Ambion) à partir du plasmide pGb-Eg2-410Δ2-hxG-A65 linearisé avec *Eco*RV (Legagneux V, Omilli F, Osborne HB (1995) Substrate-specific regulation of RNA deadenylation in Xenopus embryo and activated egg extracts. RNA 1: 1001-1008).

Les ARNm Luc R ont été traduits une heure à 30°C en lysat de réticulocyte de lapin (Flexi Rabbit Reticulocyte Lysate System, Promega) en présence de 50 µM de peptide. L'activité luciférase a été déterminée en utilisant le *Renillia luciferase assay* (Promega) sur le luminomètre Tristar LB941 (Berthold).

### Isolement des lymphocytes

Les lymphocytes B LLC ont été obtenus à partir de sang périphérique de 18 patients différents atteints d'une leucémie lymphoïde chronique. Les patients n'avaient pas reçu de traitement à la date du prélèvement, et présentaient différents stades d'évolution (stade A, 12 patients; stade B, 3; stade C, 3) selon la classification de Binet. Les prélèvements ont été réalisés dans le centre hospitalier de Brest, au service d'hématologie. Les prélèvements sanguins ont été effectués après information et obtention du consentement avisé des patients.

Les lymphocytes B et les lymphocytes T contrôles proviennent de prélèvement de sang périphérique de patients atteints d'hémochromatose, fournies par le centre de transfusion CTS à BREST. Les lymphocytes B et T ont été isolés à l'aide du kit Rosett-Sep (StemCell Technologies) selon les recommandations du fournisseur.

Les lymphocytes, cellules mononuclées, ont été isolées à partir des prélèvement de sang périphérique héparinés par centrifugation Ficoll-Hypaque (LSM 1077 lymphocyte, euro-bio). Les globules rouges ont été lysés par 10 mn d'incubation dans du VersaLyse Lysing Solution (Beckman Coulter). Les cellules mononuclées sont obtenues après 2 lavages dans du milieu de culture (RPMI1640, Gibco BRL).

Après isolement, la pureté des lymphocytes B a été analysée par détection des antigènes CD5,et CD19 en cytométrie de flux (paragraphe 5)

### Culture cellulaire

Les cellules ont été cultivés dans un milieu de culture composé soit de RPMI 1640 (lymphocytes B) soit de DMEM (COS7, HCT116, HGT1, 293, HeLa) contenant 10% de FBS (sérum de veau foetal, 1% de penicilline-streptomycine et 1% de glutamine, Gibco BRL). Les cellules sont distribuées en plaque 24 puits et cultivés à 37°C en atmosphère humide sous 5% de CO2.

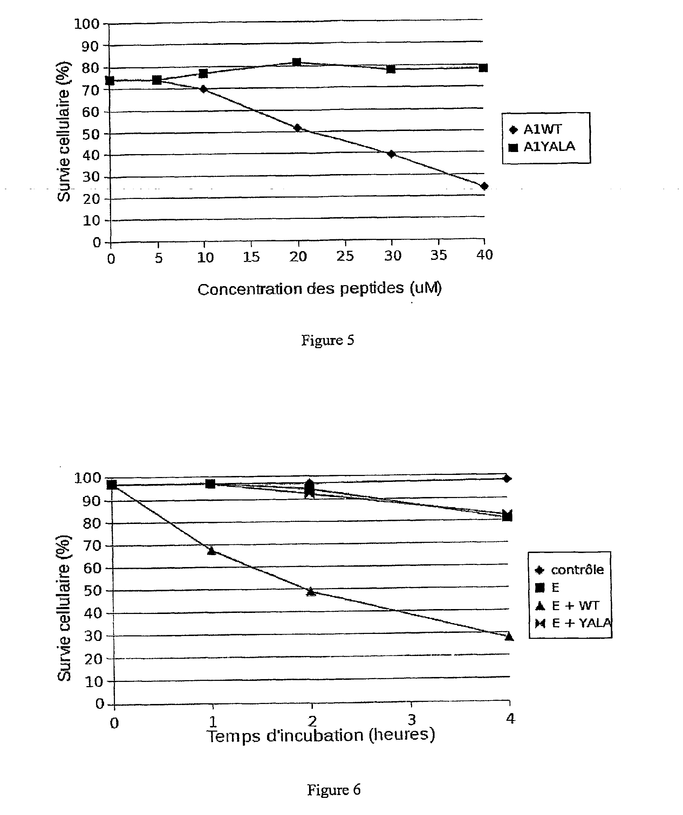

400µl de suspension cellulaire (1x10⁶ cellules/ml) par puit ont été traitées pendant 4h ou 24h avec différentes concentrations de peptides. Le test d'activité des peptides en présence d'émétine (Figure 6) a été réalisé en préincubant les cellules avec 100 µM d'émétine pendant une heure avant ajout de peptide.

### Analyse en cytométrie de flux.

Les analyses ont été réalisées à l'aide du cytomètre Coulter epics XL (Beckman Coulter).

### Evaluation de la pureté des lymphocytes B de LLC et des lymphocytes B sains

Pour chaque analyse en cytométrie de flux, 2 tubes distincts ont été préparés contenant chacun 2.10⁵ cellules reprises dans 100µl de tampon PBS. Dans un des deux tubes, 10 µl d'anticorps anti-CD19 couplés au fluorochrome Phycoerythine (PE)(CD19 PE, Beckman Coulter) et d'anticorps anti-CD5 couplés au fluorochrome Phycocyanine (PC)(CD5 PC, Beckman Coulter) sont ajoutés. Dans le second tube utilisé comme contrôle, sont ajoutés 10µl d'anticorps d'isotypes aspécifiques couplés aux fluorochromes PE (IgG1PE, Beckman Coulter) et PC (IgG1PCy5, Beckman Coulter). Ces anticorps fixent les marqueurs de surface autres que CD5+ et CD19+. Par conséquent, le second tube sert de contrôle négatif dans l'analyse des données en cytométrie de flux. Les deux tubes sont incubés 10 minutes à l'obscurité à température ambiante. A la fin de l'incubation est ajouté 1ml de PBS additionné de BSA à 5%, puis les cellules sont lavées comme précédemment (centrifugation 1200 rpm, 10 minutes, 20°C). A la suite de ce lavage, le surnageant est enlevé puis 500µl de PBS BSA 5% sont rajoutées sur le culot cellulaire.2.10⁵ cellules dans 100µl de PBS ont été exposées aux anticorps antiCD5PC5 et anti CD19PE couplés à des fluorochromes pendant 10 minutes à l'obscurité. La fluorescence des cellules est analysée après lavage au PBS.

### Mesure du pourcentage de la survie cellulaire

Après lavage des cellules au PBS, les cellules ont été analysées à l'aide du système Annexin V-FITC IP selon les recommendations du fournisseur (Beckman Coulter).

### Analyses par microscopie à fluorescence

Les analyses ont été réalisées sur un microscope à épifluorescence Zeiss Observer Z1. Les cellules sont cultivées en plaque multipuits à fond de verre, de l'iodure de propidium et du Syto13 (Invitrogen) sont ajoutés dans le milieu à une concentration respectivement de 1 mg/ml et de 5 µM. Le Syto 13 se lie à l'ADN au niveau des noyaux des cellules qui émettent alors une fluorescence verte. Lorsque la perméabilité membranaire est modifiée au cours du processus de mort cellulaire, l'iodure de propidium pénètre dans la cellule et provoque l'apparition d'une fluorescence rouge lors de sa liaison à l'ADN des noyaux des cellules. Le rapport entre le nombre de noyaux émettant une fluorescence verte et le nombre de noyaux émettant une fluorescence rouge représente la fraction de cellules vivantes exprimées en pourcentage de survie cellulaire dans les figures.

### Exemple 1 : Identification de peptides de fortes affinités avec eIF4E

Afin d'identifier et de caractériser des peptides à très forte affinité pour eIF4E, les inventeurs ont développé une approche de génomique fonctionnelle originale appelée 3D-S, qui combine biologie structurale et bio-informatique.

Différentes études de l'interaction entre eIF4E et ses partenaires ont montré qu'une stratégie de liaison commune était utilisée. Le motif de reconnaissance d'eIF4E est défini par Tyr-X-X-X-X-Leu-Φ (SEQ ID No : 25) (où X est variable et Φ est un résidu hydrophobe, à savoir Leu, Met ou Phe). Pour trouver de nouveaux partenaires d'eIF4E, il a été recherché des protéines possédant ce motif dans une base de données contenant des protéines de nombreuses espèces : Trembl (http://www.ebi.ac.uk/trembl). Un nombre très important de protéines contiennent ce motif, ce qui est incompatible avec le nombre attendu de partenaires pour eIF4E considérant ses rôles spécifiques. Ce motif commun aux partenaires d'eIF4E connus est donc minimal mais n'est pas suffisant pour définir un site de liaison à eIF4E.

Les inventeurs ont alors tiré profit des structures cristallographiques disponibles pour comprendre le mode d'interaction d'eIF4E et de ses partenaires, afin de construire une matrice de substitution d'acides aminés pour le site de liaison à eIF4E. En d'autres termes, il s'agit de définir une matrice d'acides aminés englobant l'ensemble des séquences dont la structure 3D est compatible avec une interaction avec eIF4E.

Sur la base de l'observation en 3D des structures d'eIF4E en interaction avec un peptide issu de 4E-BP ou d'un peptide issu de eIF4G, l'effet de changement d'acide aminé a été testé pour chaque position du site de liaison à eIF4E, sur une longueur de 10 acides aminés. Ceci a permis de générer une matrice d'analyse, dénommée matrice 3D-S/4EBS (matrice d'analyse 3D-S pour eIF4E binding site).

A l'aide de cette matrice d'acides aminés, de nouveaux partenaires d'eIF4E ont été recherchés par bio-informatique. Comme attendu, eIF4G et 4EBP ont été trouvés. Ceci a permis d'identifier également 582 protéines possédant cette séquence (sur un total de 2914826 protéines).

Parmi les protéines identifiées, des faux négatifs peuvent correspondre à des protéines qui ne présentent par le site d'interaction à leur surface, ou qui sont localisées dans des compartiments cellulaires sans eIF4E. Afin d'augmenter la spécificité de cette recherche, les inventeurs ont posé l'hypothèse que si l'interaction d'eIF4E avec un partenaire était importante fonctionnellement, le motif devait être conservé dans l'évolution. La base de données comportant des protéines de nombreuses espèces, une recherche de protéines homologues parmi les 582 protéines issues de la première sélection a été réalisée. Cette analyse d'homologie a consisté en l'alignement des protéines 2 à 2 et à la constitution de groupes de protéines en prenant les paramètres suivants : la taille de l'alignement entre 2 séquences A et B doit correspondre à au moins 75% de longueur de A, et le pourcentage d'identité doit être supérieur à 50%.

Parmi les 582 protéines issues de la première sélection, 315 ont été classée en 66 groupes. Quatorze groupes ont été jugés ples plus intéressants. Ces groupes, rassemblant 152 protéines au total, sont constitués de protéines dont plus de 2 homologues possèdent le site de liaison à eIF4E. L'un des groupes comporte même des homologues issus de 10 espèces différentes. Parmi ces 14 groupes, 6 correspondent à des protéines annotées, représentées dans des banques d'ADNc, mais pour lesquelles aucune fonction n'a été décrite.

En conclusion, le criblage 3D-S de la base de données de protéines Trembl (http://www.ebi.ac.uk/trembl) a permis de sélectionner 582 protéines (possédant une séquence 4EBP like) classées en 14 groupes.

La validation biochimique des peptides identifiés a ensuite été effectuée par des approches de chromatographie d'affinité. La séquence 4EBPlike de l'homologue humain de chacun des 14 groupes a été utilisée pour produire des protéines de fusion entre YFP *(yellow fluorescent protein)* et chaque peptide en lysat de réticulocytes de lapin (milieu réactionnel permettant la traduction d'ARNm *in vitro).* La capacité de ces peptides à interagir avec eIF4E a été ensuite analysée par chromatographie d'affinité. Parmi les 14 protéines testées, 6 interagissent avec eIF4E. De manière intéressante, un peptide appelé YFP-A1 WT (protéine YFP, yellow fluorescent protein, fusionnée à un peptide issu de la protéine *Angel 1* humaine, de séquence RRKYGRDFLLRF (SEQ ID No : 3)), s'associe plus fortement à eIF4E que les autres peptides testés.

### Exemple 2 : le peptide A1 WT interagit avec eIF4E, la mutation YALA abolit cette interaction.

La séquence 4E-BP like de la protéine A1 sauvage *(wild type,* WT) fusionnée à la protéine YFP et la protéine YFP seule utilisée comme contrôle ont été produites en lysat de réticulocyte de lapin et marquées à la méthionine radioactive (Met ³⁵S). Les protéines YFP-A1WT et YFP ont ainsi été obtenues sous forme soluble. Trois acides aminés connus pour être essentiels à l'interaction avec eIF4E dans la séquence de 4E-BP2 humaine sont retrouvés dans la séquence 4E-BP like de la protéine A1 sauvage. Afin d'obtenir une protéine mutante incapable d'interagir avec eIF4E utilisable comme contrôle, ces trois acides aminés ont été mutés pour obtenir la protéine YFP-A1 YALA (mutation des acides aminés tyrosine (Y) et leucine (L) en alanine (A)), dans lequel le peptide A1 YALA présente la séquence suivante : RRKAGRDFAARF (SEQ ID No : 5).

Les protéines produites ont été analysées sur un gel SDS-PAGE suivi d'une autoradiographie (Figure 1, "input 1/10", pistes 1, 4, 7). YFP- A1 WT est retenue sur des billes GST-eIF4E (piste 5) alors que YFP non fusionnée n'est pas retenue (piste 2). Les billes GST ne retiennent aucune protéine, démontrant la spécificité de l'interaction (piste 6). Lorsque les trois acides aminés sont mutés l'interaction est très faible (piste 8). Cette expérience montre l'interaction entre eIF4E et la séquence 4E-BP like de la protéine Angel 1, et que cette interaction est perdue après mutation YALA.

### Exemple 3 : le peptide A1WT,possédant un site d'interaction avec eIF4E, inhibe la traduction

Le peptide correspondant à la séquences 4E-BP like de la protéine Angel 1 (A1WT), a été synthétisé chimiquement en fusion avec la séquence IRS composée des acides aminés RYIRS (SEQ ID No: 4) ; pour une utilisation *in vivo,* cette séquence peut soit être conservée, soit être remplacée par une autre séquence pénétratine, par un autre polypeptide tel qu'une molécule du CMH, par exemple HLA-DR, ou par un autre élément, tel qu'un carbohydrate ou une nano-particule, *etc..* Des peptides déjà connus pour interagir avec eIF4E issus des protéines 4E-BP2 humaine (BP2WT) et 4E-BP d'oursin *Sphaerichinus granularis* (SgWT) ont également été synthétisés, également fusionnés à la séquence IRS. De plus, les acides aminés tyrosine (Y) et leucine (L), connus comme étant essentiels à l'interaction avec eIF4E pour la protéine 4E-BP2 humaine, ont été remplacés par des alanines (A) pour obtenir les peptides A1 YALA IRS, BP2 YALA IRS et Sg YALA IRS qui ont été utilisés comme contrôles. La pureté des peptides analysée par spectrométrie de masse est supérieure à 95%.

| | |
|---|---|
| A1 YALA IRS : | RRKAGRDFAARFRYIRS (SEQ ID No : 6) |
| BP2 WT IRS : | RIIYDRKFLLDRRYIRS (SEQ ID No : 7) |
| BP2 YALA IRS : | RIIADRKFAADRRYIRS (SEQ ID No : 8) |
| BPSg WT IRS : | RIIYDRHFLLNMRYIRS (SEQ ID No : 9) |
| BPSg YALA IRS : | RIIADRHFAANMRYIRS (SEQ ID No : 10) |

Un ARNm coiffé (possédant une coiffe appelée "cap" à l'extrémité 5') codant pour la luciférase, protéine facilement dosable par la mesure de son activité, a été traduit en présence de ces différents peptides en lysat de reticulocytes de lapin (Figure 2). L'activité luciférase mesurée est directement proportionnelle à la quantité de protéine luciférase produite et donc à l'activité traductionnelle dépendante de la coiffe (*"cap-dependent").* Les peptides étant dissous dans du DMSO, il était important de tester l'effet du DMSO sur la traduction. Il a été observé que l'ajout de DMSO n'affectait pas la traduction (comparer l'ajout d'eau (H2O) et de DMSO, figure 2). L'activité traductionnelle en présence de peptide a été comparée à celle obtenue lors de l'ajout d'un volume égal de DMSO, considérée égale à 100%. En présence du peptide BP2 WT IRS à une concentration finale de 50 µM, une forte inhibition de la traduction a été observée par rapport au contrôle. Par contre le peptide BP2 YALA IRS utilisé comme contrôle négatif (il n'interagit pas avec eIF4E), ne provoque pas d'inhibition de la traduction de la luciférase. Il en est de même pour les peptides issus de la séquence de 4E-BP de l'oursin (BPSg WT IRS et BPSg YALA IRS). De manière intéressante, le peptide issu de la protéine Angel 1 (A1 WT IRS) inhibe la traduction de façon plus importante, comparé aux autres peptides. Comme attendu, le peptide A1 YALA IRS n'inhibe pas la traduction démontrant la spécificité d'action du peptide A1 WT IRS sur la traduction dépendante d'eIF4E.

### Exemple 4 : effet des peptides sur des lymphocytes LLC prélevés chez des patients puis mis en culture

Les cellules prélevées sur des patients atteints de leucémie lymphoïde chronique ont été purifiées sur coussin de Ficoll. La pureté des lymphocytes B analysée par détection des antigènes CD5 et CD19 par FACS est supérieure à 95% dans toutes les expériences réalisées. Les cellules ont été mises en culture dans du milieu RPMI en présence de 10% FBS (sérum de veau foetal). Les peptides ont été ajoutés directement dans le milieu de culture. Ces peptides comportent la séquence IRS (dont la séquence en acides aminés est RYIRS) qui est connue pour permettre la pénétration de peptides dans les lymphocytes B en culture (Dong et al., 2005). Les cellules ont été analysées avec le kit de détection annexin/Iodure de Propidium (Beckman Coulter), permettant de mettre en évidence l'exposition extracellulaire de phosphatidylsérine et l'apparition d'une perméabilté membranaire, événements caractéristiques de la PCD. Les cellules qui survivent au traitement sont celles qui ne sont marquées ni par l'annexine ni par l'Iodure de Propidium. Les conditions de culture permettent la survie des lymphocytes purifiés puisque le pourcentage de survie ne passe que de 96 à 84% après 4H et 24H de culture respectivement (Figure 3). Lorsque les peptides 4EBP2WT et BPSgWT (connus pour interagir avec eIF4E) ont été incubés avec les cellules, le pourcentage de survie n'a pas diminué. Par contre le peptide A1WT (peptide découvert au cours de cette étude) a provoqué une forte mortalité puisque seulement 25% des cellules survivaient après 4H d'incubation. Ce pourcentage est passé à 19% après 24H, indiquant que l'effet du peptide est essentiellement précoce. Le peptide A1 YALA IRS, incapable d'interagir avec eIF4E, n'a pas d'effet, ce qui indique que l'activité du peptide A1 WT IRS est spécifique. Cette expérience permettant de comparer les effets des peptides 4EBPWT, BPSgWT et A1WT a été vérifiée sur 3 lots de cellules purifiées (obtenues à partir de 3 patients différents atteints de LLC), et sur 2 lots de peptides A1 (pureté supérieure à 95 et 99% respectivement pour chaque lot).

Afin de suivre l'évolution de l'effet du peptide A1WT au cours des 24H de l'expérience, le pourcentage de survie lors de l'incubation avec les peptides A1 WT IRS et A1 YALA IRS a été analysé à différents temps après incubation. Les résultats sont présentés figure 4. Alors que l'effet du peptide A1 WT IRS est observé au cours des 4 premières heures d'incubation, la différence de survie par rapport aux cellules non traitées n'augmente plus ensuite, ce qui indique que le peptide n'est plus actif après 4H d'incubation. Ceci peut s'expliquer par une dégradation du peptide au cours de l'incubation dans le milieu de culture. En conclusion, le peptide A1WT provoque une forte mortalité des lymphocytes B LLC, et cet effet est essentiellement précoce au cours de l'incubation avec les cellules en culture.

Lorsque le peptide A1 WT IRS a été incubé à différentes concentrations avec les cellules LLC pendant 24H, la mortalité cellulaire a augmenté avec la concentration de peptide (figure 5). L'IC50, définie comme la concentration de peptide capable d'entraîner la mort de 50% des cellules après 24H est d'environ 28 µM. Cette expérience a été reproduite sur un lot de cellules provenant d'un deuxième patient, pour lequel l'IC50 s'est élevée à 24 µM.

eIF4E, cible du peptide A1WT, intervient dans la régulation de l'initiation de la traduction, mais également dans la stabilité et le transport des ARNm. Les inventeurs ont voulu déterminer si la traduction était nécessaire à l'effet du peptide (figure 6). L'ajout d'émétine, inhibiteur de la traduction, n'a provoqué qu'un faible effet sur la survie des cellules, qui est passée de 97 à 82% après 4H d'incubation. L'efficacité de l'inhibition de la traduction a été vérifiée par incorporation de Methionine 35S (résultat non présenté). Lors de l'ajout du peptide A1WT en présence d'émétine, la survie des cellules est passée de 97% à 27% en 4H, ce qui montre que le peptide est toujours efficace en absence de traduction. Cette expérience reproduite sur un deuxième lot de cellules purifiées a conduit à un résultat similaire. L'effet du peptide A1WT sur eIF4E ne concerne donc pas directement ses fonctions de régulation de la traduction.

Ainsi, parmi les différents peptides 4EBP-like testés qui interagissent avec eIF4E, et inhibent la fonction d'eIF4E dans la traduction, seul le peptide A1WT identifié au cours de cette étude provoque, par son interaction avec eIF4E, la mort cellulaire de lymphocytes B de patients atteints de LLC. Cet effet est indépendant de la traduction et touche donc une fonction d'eIF4E indépendante de la traduction.

Le peptide A1WT est donc un peptide original potentiellement exploitable en thérapie contre la LLC.

### Exemple 5 : Effet du peptide A1 sur différentes lignées cellulaires d'origine myéloïde, lymphoïde, gastrique, colique ou rénale.

Afin de déterminer si le peptide A1 pouvait être potentiellement exploitable pour la thérapie d'autres pathologies que la LLC, les inventeurs ont testé l'effet du peptide A1 WT IRS sur d'autres cellules d'origine lymphoïde ou myéloïde. Le peptide A1 YALA IRS est utilisé comme contrôle.

Les cellules d'origine lymphoïde provenaient toutes de prélèvements chez des patients souffrant de différentes pathologies classées en fonction du siège de la prolifération tumorale. Cette prolifération a lieu soit dans la moelle osseuse, pour la LLC, la Leucémie pro-lymphocytaire B et la leucémie aigüe lymphoblastique T (LAL T); soit dans les ganglions pour le lymphome de manteau et le lymphome de la zone marginale. Un classement peut également être fait en fonction du type cellulaire touché : lymphocytes B (LLC, Leucémie pro-lymphocytaire B, lymphome de manteau et lymphome de la zone marginale) ou lymphocytes T (LAL T).

Les cellules d'origine myéloïde ont soit été prélevées sur des patients atteints de leucémie aiguë myéloïde (2 patients).

Les inventeurs ont d'abord testé une autre lignée leucémique que la LLC : la leucémie pro-lymphocytaire B (pro-ly, fig.7). Comme indiqué figure 7, ces cellules sont sensibles au peptide A1WT puisque la survie cellulaire n'est que de 20% après 24H de traitement, proche du taux de survie observé pour les cellules LLC. Le peptide A1WT peut donc toucher des cellules issues de différentes lignées leucémiques dont la LLC et la leucémie pro-lymphocytaire B.

Des cellules cancéreuses de type B mais non leucémiques et provenant de lymphomes malins dont l'origine prédominante est ganglionnaire ont ensuite été testées. Il s'agit du lymphome du manteau (MAN, fig.7, 1 patient) et du lymphome de la zone marginale (LZM, 1 patient). Le taux de survie en présence du peptide A1WT est d'environ 30% (Fig. 7), proche de celui observé pour les cellules LLC. L'activité du peptide A1WT n'est donc pas spécifique de cellules issues de pathologies leucémiques.

L'effet du peptide A1WT a ensuite été testé sur des lymphocytes T obtenus par prélèvement d'un patient atteint d'une leucémie aiguë lymphoblastique T (LALT, Fig. 7). Les résultats montrent que l'effet du peptide est aussi important que pour les cellules LLC. Le peptide agit donc sur des cellules cancéreuses de type T comme de type B.

Une lignée myéloïde a ensuite été utilisée. Le peptide A1WT est actif sur des cellules prélevées chez deux patients atteints de leucémie aiguë myéloïde (LAM, Fig. 7) puisque qu'un taux de survie de 38% est observé. Ces résultats montrent que le peptide n'est pas spécifique des pathologies lymphoïdes mais est également actif sur les lignées myéloïdes testées.

L'effet du peptide a également été testé sur des lymphocytes B et T (L.T et L.B, Fig. 7) obtenus à partir de sang périphérique prélevés chez 6 patients souffrant d'une maladie non liée à une pathologie lymphocytaire, l'hémochromatose. Les résultats ont montré que le peptide agit également sur ces lymphocytes B et T, bien que l'effet était plus important sur les lymphocytes B que sur les lymphocytes T avec un taux de survie respectivement de 12% et 38% après 4H d'incubation avec 40 µM de peptide A1WT (Figure 7).

Il est intéressant de noter que le peptide agit sur des cellules bloquées en G0-G1 (lymphocytes B provenant de patient atteint de LLC) et également sur les cellules qui sont en prolifération (toutes les lignées utilisées sauf lymphocytes B provenant de patient atteint de LLC).

Le peptide étant efficace sur de nombreuses pathologies cancéreuses hématopoïétiques, les inventeurs ont ensuite testé des cellules issues d'un cancer solide, la lignée cellulaire HGT1 issue d'un cancer gastrique. L'effet du peptide était faible, puisque plus de 88% des cellules survivent après 4H de traitement avec le peptide A1WT (HGT1, fig.7). De même, la survie de cellules issues d'un cancer colique humain ou d'un cancer du rein simien est peu affectée par le peptide (respectivement 94 et 90% de survie cellulaire). Toutefois, les résultats obtenus sur les cellules issues d'un cancer colique humain ou d'un cancer du rein peuvent être dus à la technologie utilisée, à savoir la cytométrie de flux, qui est bien adaptée pour les cellules en suspension mais moins pour les cellules adhérentes (comme les cellules des lignées COS7, HCT116 et HGT1) qui doivent être décollées pour analyse, les cellules mortes pouvant être perdues lors des lavages et ne pas être comptabilisées.

Les inventeurs ont donc complété ces expériences en utilisant une technique de marquage de cellules identique (à savoir le test de perméabilité à l'iodure de propidium) mais en utilisant une approche en microscopie à fluorescence, qui permet de quantifier la mortalité cellulaire directement et de réaliser des cinétiques fiables. Ainsi, des cellules issues de la transformation par l'adénovirus 5 de cellules de rein d'embryon humain (293) et dérivées d'un cancer du col de l'utérus (HeLa) ont été testées. Les résultats montrent un effet dose dépendant du peptide sur les deux lignées testées, avec une sensibilité élevée des cellules HeLa, comparable à celle observée pour les lymphocytes B issues de patients atteints par la leucémie lymphoïde chronique (JOK) (Fig. 10). L'efficacité du peptide n'est donc pas limitée aux cellules hématopoïétiques.

De plus, les inventeurs ont observé que l'effet du peptide est extrêmement rapide, puisque dès 15 min après l'ajout du peptide, le taux de survie des lymphocytes B issues de patients atteints par la leucémie lymphoïde chronique (JOK) passe de 98 à 70% (Fig. 11).

Afin de classer les cellules en fonction de leur sensibilité au peptide, les cellules ont été exposées à différentes concentrations de peptide A1 WT IRS (Fig. 8). Pour toutes les cellules analysées, le taux de survie cellulaire diminue lorsque la concentration en peptides augmente.

En termes de taux de réponse au peptide, on peut définir 3 groupes de populations cellulaires, en fonction du taux de survie cellulaire par rapport aux cellules incubées sans peptide, après 4h d'incubation avec 20µM de peptide :
- Cellules répondant faiblement, survie cellulaire supérieure à 90% : Leucémie Aigüe Myéloïde (LAM) et cellules issues de cancers gastrique, colique ou rénal.
- Cellules présentant une réponse partielle, survie cellulaire comprise entre 75 et 90% : lymphome de la zone marginale (LZM), lymphome du manteau, lymphocytes T contrôles.
- Cellules présentant une réponse importante, survie cellulaire inférieure à 65% : LLC, Leucémie pro-lymphocytaire B, lymphocytes B contrôles, leucémie aigüe lymphoblastique T (LALT).

En conclusion, le peptide A1WT pourrait ouvrir des perspectives de thérapie de différentes pathologies hématopoïétiques.

### Exemple 6 : analyse de l'effet de différents mutants obtenus à partir du peptide A1 sur des lymphocytes LLC prélevés chez des patients

Les inventeurs ont ensuite réalisé une série de peptides variants, obtenus par substitution (conservative ou non) d'un ou deux acides aminés du peptide A1, ou par délétion des deux premiers acides aminés. La liste des peptides testés dans cette nouvelle série d'expériences est indiquée ci-après :

**Tableau 1 : Présentation de la séquence des peptides et de leur effet cellulaire. 1 ère colonne :**

| | | | Effet cellulaire | | | |
|---|---|---|---|---|---|---|
| Peptide | Séquence | SEQ ID No | LLC 40 | LT 40 | Jok 20 | Jok 40 |
| A1 WT IRS | RRKYGRDFLLRFRYIRS | 2 | + | + | + | + |
| A1 YALA IRS | RRKAGRDFAARFRYIRS | 6 | - | - | - | - |
| A1 K3I IRS | RRIYGRDFLLRFRYIRS | 11 | - | nd | +/- | + |
| A1 del2 IRS | KYGRDFLLRFRYIRS | 12 | - | - | +/- | + |
| A1 R1,2K IRS | KKKYGRDFLLRFRYIRS | 13 | + | +/- | +/- | + |
| A1 R1,2G IRS | GGKYGRDFLLRFRYIRS | 14 | - | - | - | +/- |
| A1 K3R IRS | RRRYGRDFLLRFRYIRS | 15 | + | + | + | + |
| A1 G5A-IRS | RRKYARDFLLRFRYIRS | 16 | + | + | + | + |
| A1 G5S IRS | RRKYSRDFLLRFRYIRS | 17 | + | + | + | + |
| A1 R6K IRS | RRKYGKDFLLRFRYIRS | 18 | + | +/- | +/- | + |
| A1 R6H IRS | RRKYGHDFLLRFRYIRS | 19 | +/- | +/- | + | + |
| A1 D7E IRS | RRKYGREFLLRFRYIRS | 20 | + | + | + | + |
| A1 D7A IRS | RRKYGRAFLLRFRYIRS | 21 | + | + | + | + |
| AI F12K IRS | RRKYGRDFLLRKRYIRS | 22 | - | - | - | - |
| A1 F12W IRS | RRKYGRDFLLRWRYIRS | 23 | + | + | + | +/- |
| BP2 WT IRS | RIIYDRKFLLDRRYIRS | 7 | - | nd | - | - |
| BP2 I3K IRS | RIKYDRKFLLDRRYIRS | 24 | - | - | - | - |

nom des peptides. 2^{ème} colonne : séquence des peptides, les acides aminés mutés par rapport à la séquence de A1 WT IRS sont indiqués en gras. Le peptide A1 YALA IRS correspond aux mutations Y4AL9,10A dans la séquence A1 WT IRS. 3^{ème} colonne : numéro des séquences. Colonnes suivantes : niveau d'effet des peptides pour induire la mortalité de lymphocytes B de patients LLC à 40 µM (colonne LLC 40), de lymphocytes T contrôles à 40 µM (colonne LT40), de cellules Jok à 20 (colonne Jok 20) ou à 40 µM (colonne Jok 40). + : survie < 25 %, +/- : survie comprise entre 25 et 50%, - : survie > 50%, n.d : non déterminé ; ce pourcentage de survie cellulaire correspond au pourcentage de cellules non marquées (ni par l'annexine ni par l'iodure de propidium), évalué par cytométrie de flux, après 4 heures de culture en présence de peptide. L'échantillon contrôle correspond aux cellules incubées sans peptide.

Les peptides ont été testés sur des lymphocytes B de patients atteints de leucémie lymphoïde chronique, des lymphocytes T contrôles, ou des cellules Jok (lignée cellulaire dérivée de lymphocytes B). L'expérience a été répétée 3 fois pour chaque type de cellule testé.

Les cellules incubées sans peptide ont présenté un pourcentage de survie supérieur à 96% (contrôle, figure 9).

Le peptide A1 WT IRS (SEQ ID No : 2) induit à 40 µM une forte mortalité cellulaire dans les cellules LLC et les cellules Jok (figure 9 et tableau 1) ; le taux de survie est plus élevé dans les cellules T contrôles comme déjà décrit dans les expériences précédentes (figures 7 et 8).

Le peptide A1 YALA IRS (SEQ ID No : 6, correspondant à Y4AL9AL10A A1 IRS), utilisé comme contrôle négatif puisqu'il ne se lie pas à eIF4E, comme attendu n'a pas d'effet.

Le peptide A1 del2 IRS (SEQ ID No : 12) : la délétion des deux premiers acides aminés de la séquence de A1 WT IRS provoque la perte d'effet du peptide à 40 µM sur les cellules LLC, les lymphocytes T contrôles, ainsi que sur les cellules Jok à 20 µM. A 40 µM, il provoque tout de même une mortalité importante sur les cellules Jok.

Le peptide A1 R1,2K IRS (SEQ ID No : 13): le remplacement des deux arginines (RR) par 2 acides aminés lysine (KK) diminue l'effet à 40 µM dans les cellules LLC et les lymphocytes T contrôle, ainsi que sur les cellules Jok à 20 µM. A 40 µM, il a le même effet que le peptide A1 WT IRS sur les cellules Jok.

Le peptide A1 R1,2G IRS (SEQ ID No : 14): La substitution des deux premiers acides aminés par 2 acides aminés neutres glycine (GG) abolit l'effet du peptide, sauf dans les cellules Jok lorsqu'il est utilisé à 40µM où son effet n'est que partiellement diminué.

Les peptides A1 K3R IRS (SEQ ID No : 15) et A1 K3I IRS (SEQ ID No : 11): la substitution de l'acide aminé lysine en position 3 par une arginine (R) ne modifie pas l'effet du peptide dans les trois types cellulaires, alors que la substitution du même acide aminé par une isoleucine (I) provoque la perte d'effet du peptide à 40 µM sur les cellules LLC, ainsi que partiellement sur les cellules Jok à 20 µM.

Les peptides A1 G5A IRS (SEQ ID No : 16) et A1 G5S IRS (SEQ ID No : 17) : la substitution de l'acide aminé glycine (G) en position 5 par un acide aminé alanine (A) ou par une serine (S) augmente légèrement l'effet du peptide dans les trois types cellulaires.

Les peptides A1 R6K IRS (SEQ ID No : 18) et A1 R6H IRS (SEQ ID No : 19) : la substitution de l'acide aminé R (Arginine) en position 6 par l'acide aminé lysine (K) diminue partiellement l'action de peptides dans les trois types cellulaire alors que sa substitution par une histidine (H) annule l'effet sur les cellules LLC et les lymphocytes T contrôles, contrairement aux cellules Jok où l'effet est potentialisé.

Les peptides A1 D7E IRS (SEQ ID No : 20) et A1 D7A IRS (SEQ ID No : 21) : la substitution de l'acide aminé acide aspartique en position 7 par des acides aminés acide glutamique (E) ou alanine(A) ne modifie pas l'action de peptides dans les cellules LLC et les lymphocytes T saines, sauf pour le peptide D7E dans les cellules de Jok où sont effet est potentialisé.

Le peptide A1 F12K IRS (SEQ ID No : 22) et A1 F12W IRS (SEQ ID No : 23) : la substitution de l'acide aminé phenylalanine (F) en position 12 par l'acide aminé lysine (K) abolit complètement l'action du peptide dans les trois types cellulaires, alors que son remplacement par un tryptophane (W) ne modifie pas l'action de peptides dans les trois types cellulaires.

Le peptide BP2 WT IRS (SEQ ID No : 7) correspond à la séquence de liaison à eIF4E de la protéine 4E-BP2 humaine. 4E-BP2 est une protéine connue pour interagir avec eIF4E (choisie pour son affinité pour eIF4E qui est supérieure à 4E-BP1 (Ptushkina, EMBO J, 1999)). Ce peptide n'a pas d'effet ni sur les cellules LLC ni sur les cellules Jok.

Le peptide BP2 I3K IRS (SEQ ID No : 24) : il s'agit de la séquence BP2 mutée en position 3. Une isoleucine est présente en position 3 de BP2 et SBP, alors qu'une lysine est présente à cette position dans la séquence de A1 WT. Les inventeurs ont testé si cette différence pouvait être à l'origine de la différence d'effet cellulaire entre A1 WT et BP2 WT. Les résultats montrent que le substitution de l'acide aminé isoleucine (I) en position 3 de BP2WT par l'acide aminé lysine (K) ne confère pas à ce dernier une meilleure efficacité.

Alors que le peptide BP2 inhibe efficacement la traduction dépendante de eIF4E (fig. 2), il n'a pas d'effet sur la survie cellulaire. La capacité d'un peptide de se lier à eIF4E et d'inhiber la traduction n'est donc pas suffisante pour entraîner la mortalité cellulaire observée avec A1WT.

Par ailleurs, les inventeurs ont synthétisé un peptide A1 G5AD7A IRS (SEQ ID No : 30) présentant à la fois les substitutions G5A et D7A mentionnées ci-dessus. L'action du peptide est similaire à celle obtenue pour A1 G5A IRS et A1 D7A IRS.

En conclusion, les mutations du peptide peuvent diminuer ou augmenter l'efficacité du peptide à induire la mort cellulaire, et ceci de façon dépendante du type cellulaire analysé. En particulier :
a) Les 2 premiers acides aminés sont nécessaires à l'activité du peptide, et doivent être de préférence des acides aminés basiques, par exemple des arginines.
b) L'acide aminé en position 3 doit également être choisi parmi les acides aminés basiques (R, K, H).
c) Une sérine ou une alanine sera choisie de préférence en position 5.
d) En position 6 sera choisi de préférence une arginine (R). Une histidine sera préférée pour un moindre effet LLC et lymphocytes T contrôles par rapport aux cellules Jok.
e) La mutation testée en position 7 n'a pas d'effet. Si un moindre effet sur les Jok est recherché par rapport à l'effet sur les cellules LLC et lymphocytes T contrôles, l'acide glutamique (E) sera évité.
f) L'acide aminé en position 12 sera soit la phénylalanine (F) soit le tryptophane (W).

### REFERENCES

Boumsell, L., Bernard, A., Lepage, V., Degos, L., Lemerle, J. and Dausset, J. (1978) Some chronic lymphocytic leukemia cells bearing surface immunoglobulins share determinants with T cells. Eur J Immunol, 8, 900-904.
Caligaris-Cappio, F. and Hamblin, T.J. (1999) B-cell chronic lymphocytic leukemia: a bird of a different feather. J Clin Oncol, 17, 399-408.
Cormier, P., Pyronnet, S., Salaun, P., Mulner-Lorillon, O. and Sonenberg, N. (2003) Cap-dependent translation and control of the cell cycle. Prog Cell Cycle Res, 5, 469-475.
Cowell, S.M., Lee, Y.S., Cain, J.P. and Hruby, V.J. (2004) Exploring Ramachandran and chi space: conformationally constrained amino acids and peptides in the design of bioactive polypeptide ligands. Curr Med Chem, 11, 2785-2798.
Culjkovic, B., Topisirovic, I., Skrabanek, L., Ruiz-Gutierrez, M. and Borden, K.L. (2005) eIF4E promotes nuclear export of cyclin D1 mRNAs via an element in the 3'UTR. J Cell Biol, 169, 245-256.
Delmer, A., Ajchenbaum-Cymbalista, F., Tang, R., Ramond, S., Faussat, A.M., Marie, J.P. and Zittoun, R. (1995) Overexpression of cyclin D2 in chronic B-cell malignancies. Blood, 85, 2870-2876.
Deshayes, S., Morris, M., Heitz, F. and Divita, G. (2008) Delivery of proteins and nucleic acids using a non-covalent peptide-based strategy. Adv Drug Deliv Rev, 60, 537-547.
Dong, C., Li, Q., Lyu, S.C., Krensky, A.M. and Clayberger, C. (2005) A novel apoptosis pathway activated by the carboxyl terminus of p21. Blood, 105, 1187-1194.
Fialkow, P.J., Najfeld, V., Reddy, A.L., Singer, J. and Steinmann, L. (1978) Chronic lymphocytic leukaemia: Clonal origin in a committed B-lymphocyte progenitor. Lancet, 2, 444-446.
Foged, C. and Nielsen, H.M. (2008) Cell-penetrating peptides for drug delivery across membrane barriers. Expert Opin Drug Deliv, 5, 105-117.
Fu, S.M., Winchester, R.J. and Kunkel, H.G. (1975) Similar idiotypic specificity for the membrane IgD and IgM of human B lymphocytes. J Immunol, 114, 250-252.
Hanasaki, K., Powell, L.D. and Varki, A. (1995) Binding of human plasma sialoglycoproteins by the B cell-specific lectin CD22. Selective recognition of immunoglobulin M and haptoglobin. JBiol Chem, 270, 7543-7550.
Herbert, T.P., Fahraeus, R., Prescott, A., Lane, D.P. and Proud, C.G. (2000) Rapid induction of apoptosis mediated by peptides that bind initiation factor eIF4E. Curr Biol, 10, 793-796.
Holcik, M. and Sonenberg, N. (2005) Translational control in stress and apoptosis. Nat Rev Mol Cell Biol, 6, 318-327.
Kanovsky, M., Raffo, A., Drew, L., Rosal, R., Do, T., Friedman, F.K., Rubinstein, P., Visser, J., Robinson, R., Brandt-Rauf, P.W., Michl, J., Fine, R.L. and Pincus, M.R. (2001) Peptides from the amino terminal mdm-2-binding domain of p53, designed from conformational analysis, are selectively cytotoxic to transformed cells. Proc Natl Acad Sci USA, 98, 12438-12443.
Koromilas, A.E., Lazaris-Karatzas, A. and Sonenberg, N. (1992) mRNAs containing extensive secondary structure in their 5' non-coding region translate efficiently in cells overexpressing initiation factor eIF-4E. Embo J, 11, 4153-4158.
Malik, D.K., Baboota, S., Ahuja, A., Hasan, S. and Ali, J. (2007) Recent advances in protein and peptide drug delivery systems. Curr Drug Deliv, 4, 141-151.
Moerke, N.J., Aktas, H., Chen, H., Cantel, S., Reibarkh, M.Y., Fahmy, A., Gross, J.D., Degterev, A., Yuan, J., Chorev, M., Halperin, J.A. and Wagner, G. (2007) Small-molecule inhibition of the interaction between the translation initiation factors eIF4E and eIF4G. Cell, 128, 257-267.
Moreau, E.J., Matutes, E., A'Hern, R.P., Morilla, A.M., Morilla, R.M., Owusu-Ankomah, K.A., Seon, B.K. and Catovsky, D. (1997) Improvement of the chronic lymphocytic leukemia scoring system with the monoclonal antibody SN8 (CD79b). Am J Clin Pathol, 108, 378-382.
Morley, S.J., Coldwell, M.J. and Clemens, M.J. (2005) Initiation factor modifications in the preapoptotic phase. Cell Death Differ, 12, 571-584.
O'Brien, S., del Giglio, A. and Keating, M. (1995) Advances in the biology and treatment of B-cell chronic lymphocytic leukemia. Blood, 85, 307-318.
Preud'homme, J.L. and Seligmann, M. (1972) Immunoglobulins on the surface of lymphoid cells in Waldenstrom's macroglobulinemia. J Clin Invest, 51, 701-705.
Richter, J.D. and Sonenberg, N. (2005) Regulation of cap-dependent translation by eIF4E inhibitory proteins. Nature, 433, 477-480.
Soares, A.F., Carvalho Rde, A. and Veiga, F. (2007) Oral administration of peptides and proteins: nanoparticles and cyclodextrins as biocompatible delivery systems. Nanomed, 2, 183-202.
Topisirovic, I., Kentsis, A., Perez, J.M., Guzman, M.L., Jordan, C.T. and Borden, K.L. (2005) Eukaryotic translation initiation factor 4E activity is modulated by HOXA9 at multiple levels. Mol Cell Biol, 25, 1100-1112.
Vrhovac, R., Delmer, A., Tang, R., Marie, J.P., Zittoun, R. and Ajchenbaum-Cymbalista, F. (1998) Prognostic significance of the cell cycle inhibitor p27Kip1 in chronic B-cell lymphocytic leukemia. Blood, 91, 4694-4700.

## Revendications

1. Peptide comprenant la séquence X₁X₂X₃YX₄X₅X₆X₇LX₈X₉X₁₀ (SEQ ID No : 1), dans laquelle :
- X₁ et X₂ représentent, indépendamment l'un de l'autre, un acide aminé choisi parmi R, K et H,
- X₃ représente un acide aminé choisi parmi R, K et H,
- X₄, X₅ et X₆ représentent n'importe quel acide aminé,
- X₇ représente un acide aminé choisi parmi F, A, V, L, I et M,
- X₈ représente un acide aminé choisi parmi L, M et F,
- X₉ représente n'importe quel acide aminé,
- X₁₀ représente un acide aminé choisi parmi F, Y, W et H,
pour le traitement d'une maladie hématopoïétique maligne.

2. Peptide selon la revendication 1, pour le traitement d'une maladie choisie parmi la leucémie lymphoïde chronique, la leucémie pro-lymphocytaire B, la leucémie aigüe lymphoblastique T, le lymphome de la zone marginale, le lymphome du manteau et la leucémie aigüe myéloïde.

3. Peptide selon la revendication 1 pour le traitement d'une maladie selon l'une quelconque des revendications 1 et 2, dans lequel :
- X₁ = X₂ = R, et/ou
- X₃ = K ou R, et/ou
- X₄ = G, A ou S, et/ou
- X₅ = R ou H, et/ou
- X₆ = D, E, A, V, L, P, M, F, I ou X, et/ou
- X₇ = F, et/ou
- X₈ = L, et/ou
- X₉ = R, et/ou
- X₁₀ = F ou W.

4. Peptide selon l'une quelconque des revendications 1 à 3 pour le traitement d'une maladie selon l'une quelconque des revendications 1 à 3, dans lequel :
- X₄ = A ou S, et/ou
- X₅ = R, et/ou
- X₆ = D ou A.

5. Peptide selon l'une quelconque des revendications 1 à 4 pour le traitement d'une maladie selon l'une quelconque des revendications 1 à 4, comprenant en outre une séquence peptidique favorisant l'entrée dudit peptide dans les cellules eucaryotes, liée à l'extrémité C-terminale de la séquence SEQ ID No.: 1.

6. Peptide selon la revendication 5 pour le traitement d'une maladie selon la revendication 5, dans lequel la séquence peptidique favorisant l'entrée dudit peptide dans les cellules eucaryotes est la séquence RYIRS (SEQ ID No : 4).

7. Peptide selon la revendication 6 pour le traitement d'une maladie selon la revendication 6, **caractérisé en ce qu'**il s'agit du peptide A1-IRS de séquence RRKYGRDFLLRFRYIRS (Seq ID No : 2).

8. Peptide selon l'une quelconque des revendications précédentes pour le traitement d'une maladie selon l'une quelconque des revendications précédentes, **caractérisé en ce qu'**il comporte en outre un ou plusieurs groupement(s) protecteur(s) aux extrémités N et/ou C-terminales.

9. Peptide selon l'une quelconque des revendications précédentes pour le traitement d'une maladie selon l'une quelconque des revendications précédentes, **caractérisé en ce qu'**il est cyclisé.

10. Composition pharmaceutique pour le traitement d'une maladie hématopoïétique maligne, comprenant un peptide tel que décrit dans l'une quelconque des revendications précédentes, associé à un vecteur.

11. Composition pharmaceutique selon' la revendication 10, pour le traitement d'un cancer choisi parmi la leucémie lymphoïde chronique, la leucémie pro-lymphocytaire B, la leucémie aigüe lymphoblastique T, le lymphome de la zone marginale, le lymphome du manteau et la leucémie aigüe myéloïde.

## Patentansprüche

1. Peptid, umfassend die Sequenz X₁X₂X₃YX₄X₅X₆X₇LX₈X₉X₁₀ (SEQ ID NO: 1), wobei
- X₁ und X₂ unabhängig voneinander Aminosäuren sind, ausgewählt aus R, K und H,
- X₃ eine Aminosäure ist, ausgewählt aus R, K und H,
- X₄, X₅ und X₆ Aminosäuren sind,
- X₇ eine Aminosäure ist, ausgewählt aus F, A, V, L, I and M,
- X₈ eine Aminosäure ist, ausgewählt aus L, M und F,
- X₉ eine Aminosäure ist,
- X₁₀ eine Aminosäure ist, ausgewählt aus F, Y, W und H,
zur Behandlung einer malignen hämatopoetischen Erkrankung.

2. Peptid nach Anspruch 1 zur Behandlung einer Erkrankung ausgewählt aus chronischer lymphatischer Leukämie, B-Prolymphozytenleukämie, akuter lymphoblastischer T-Zell-Leukämie, Marginalzonenlymphom, Mantelzelllymphom und akuter myeloischer Leukämie.

3. Peptid nach Anspruch 1 zur Behandlung einer Erkrankung nach einem der Ansprüche 1 und 2, wobei
- X₁ = X₂ = R, und/oder
- X₃ = K oder R, und/oder
- X₄ = G, A oder S, und/oder
- X₅ = R oder H, und/oder
- X₆ = D, E, A, V, L, P, M, F, I oder X, und/oder
- X₇ = F, und/oder
- X₈ = L, und/oder
- X₉ = R, und/oder
- X₁₀ = F oder W.

4. Peptid nach einem der Ansprüche 1 bis 3 zur Behandlung einer Erkrankung nach einem der Ansprüche 1 bis 3, wobei
- X₄ = A oder S, und/oder
- X₅ = R, und/oder
- X₆ = D oder A.

5. Peptid nach einem der Ansprüche 1 bis 4 zur Behandlung einer Erkrankung nach einem der Ansprüche 1 bis 4, ferner umfassend eine Peptidsequenz, die den Eintritt des Peptids in eukaryotische Zellen, die an das endständige C der Sequenz SEQ ID NO: 1 gebunden sind, begünstigt.

6. Peptid nach Anspruch 5 zur Behandlung einer Erkrankung nach Anspruch 5, wobei die Peptidsequenz, die den Eintritt des Peptids in eukaryotische Zellen begünstigt, die Sequenz RYIRS (SEQ ID NO: 4) ist.

7. Peptid nach Anspruch 6 zur Behandlung einer Erkrankung nach Anspruch 6, **dadurch gekennzeichnet, dass** es sich um das Peptid A1-IRS mit der Sequenz RRKYGRDFLLRFRYIRS (SEQ ID NO: 2) handelt.

8. Peptid nach einem der vorhergehenden Ansprüche zur Behandlung einer Erkrankung nach einem der vorhergehenden Ansprüche, **dadurch gekennzeichnet, dass** es ferner eine oder mehrere Schutzgruppen am endständigen C und/oder N umfasst.

9. Peptid nach einem der vorhergehenden Ansprüche zur Behandlung einer Erkrankung nach einem der vorhergehenden Ansprüche, **dadurch gekennzeichnet, dass** es zyklisiert ist.

10. Pharmazeutische Zusammensetzung zur Behandlung einer malignen hämatopoetischen Erkrankung, umfassend ein Peptid gemäß einem der vorhergehenden Ansprüche, das an einen Vektor assoziiert ist.

11. Pharmazeutische Zusammensetzung nach Anspruch 10 zur Behandlung einer Krebserkrankung ausgewählt aus chronischer lymphatischer Leukämie, B-Prolymphozytenleukämie, akuter lymphoblastischer T-Zell-Leukämie, Marginalzonenlymphom, Mantelzelllymphom und akuter myeloischer Leukämie.

## Claims

1. A peptide for the treatment of malignant hematopoietic disease comprising the sequence X₁X₂X₃YX₄X₅X₆X₇LX₈X₉X₁₀ (SEQ ID No: 1) wherein:
- X₁ and X₂ represent, independently of one another, an amino acid chosen from R, K and H,
- X₃ represents an amino acid chosen from R, K and H,
- X₄, X₅ and X₆ represent any amino acid,
- X₇ represents an amino acid chosen from F, A, V, L, I and M,
- represents an amino acid chosen from L, M and F,
- X₉ represents any amino acid,
- X₁₀ represents an amino acid chosen from F, Y, W and H.

2. A peptide according to claim 1 for the treatment of a disease chosen from chronic lymphoid leukaemia, B-cell pro-lymphocytic leukaemia, T-cell acute lymphoblastic leukaemia, marginal zone lymphoma, mantle cell lymphoma and acute myeloid leukaemia.

3. A peptide according to claim 1 for the treatment of a disease according to either one of claims 1 or 2, wherein: .
- X1 = X₂ = R, and/or
- X₃ = K or R, and/or
- X₄ = G, A or S, and/or
- X₅ R or H, and/or
- X₆ = D, E, A, V, L, P, M, F, I or X, and/or
- X₇ = F, and/or
- L, and/or
- X₉ = R, and/or
- X₁₀ = F or W.

4. A peptide according to any one of claims 1 to 3 for the treatment of a disease according to any one of claims 1 to 3, wherein:
- X₄ = A or S, and/or
- X₅ = R, and/or
- X₆ = D or A.

5. A peptide according to any one of claims 1 to 4 for the treatment of a disease according to any one of claims 1 to 4, further comprising a peptide sequence that promotes entry of said peptide into eukaryotic cells, linked to the C-terminal end of the sequence SEQ ID No: 1.

6. A peptide according to claim 5 for the treatment of a disease according to claim 5, wherein the peptide sequence that promotes entry of said peptide into eukaryotic cells is the sequence RYIRS (SEQ ID NO: 4).

7. A peptide according to Claim 6 for the treatment of a disease according to claim 6, **characterized in that** it is the Al-IRS peptide of sequence RRKYGRDFLLRFRYIRS (Seq ID No: 2).

8. A peptide according to any one of the preceding claims for the treatment of a disease according to any one of the preceding claims, **characterized in that** it also comprises one or more protective group(s) at the N- and/or C-terminal ends.

9. A peptide according to any one of the preceding claims for the treatment of a disease according to any one of the preceding claims, **characterized in that** it is cyclized.

10. A pharmaceutical composition for the treatment of malignant hematopoietic disease comprising a peptide as described in any one of the preceding claims associated with a vector.

11. A pharmaceutical composition according to claim 10 for the treatment of a cancer chosen from chronic lymphoid leukaemia, B-cell pro-lymphocytic leukaemia, T-cell acute lymphoblastic leukaemia, marginal zone lymphoma, mantle cell lymphoma and acute myeloid leukaemia.
